(19) Europäisches Patentamt
European Patent Office
Office européen des brevets

(11) EP 4 065 174 B1

(12) # FASCICULE DE BREVET EUROPEEN

(45) Date de publication et mention de la délivrance du brevet:
**15.11.2023 Bulletin 2023/46**

(21) Numéro de dépôt: **20862002.1**

(22) Date de dépôt: **26.11.2020**

(51) Classification Internationale des Brevets (IPC):
***A61K 49/06*** (2006.01) ***A61K 49/18*** (2006.01)
***A61K 41/00*** (2020.01) ***A61P 35/00*** (2006.01)

(52) Classification Coopérative des Brevets (CPC):
**A61K 9/0009; A61K 41/0052; A61K 49/1806; A61K 49/1836; A61K 49/1839; A61P 35/00**

(86) Numéro de dépôt international:
**PCT/FR2020/052184**

(87) Numéro de publication internationale:
**WO 2021/105620 (03.06.2021 Gazette 2021/22)**

(54) **FERROFLUIDE HUILEUX BIOCOMPATIBLE ET PROCÉDÉ DE PRÉPARATION**
BIOKOMPATIBLES ÖLIGES FERROFLUID UND HERSTELLUNGSVERFAHREN
BIOCOMPATIBLE OILY FERROFLUID AND PREPARATION PROCESS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.11.2019 FR 1913249**

(43) Date de publication de la demande:
**05.10.2022 Bulletin 2022/40**

(73) Titulaires:
- **UNIVERSITE DE BORDEAUX**
**33000 Bordeaux (FR)**
- **Institut Polytechnique de Bordeaux**
**33400 Talence (FR)**
- **Centre national de la recherche scientifique**
**75016 Paris (FR)**
- **Centre Hospitalier Universitaire de Bordeaux**
**33404 Talence Cedex (FR)**

(72) Inventeurs:
- **MORNET, Stéphane**
**33440 SAINT VINCENT DE PAUL (FR)**
- **VECCO-GARDA, Clément**
**69680 CHASSIEU (FR)**
- **CRAUSTE-MANCIET, Sylvie**
**33000 BORDEAUX (FR)**
- **SANDRE, Olivier**
**33600 PESSAC (FR)**
- **COUILLAUD, Franck**
**33600 PESSAC (FR)**
- **JEANJEAN, Pauline**
**33700 MERIGNAC (FR)**
- **GENEVOIS, Coralie**
**33200 BORDEAUX (FR)**

(74) Mandataire: **Cabinet Beau de Loménie**
**51 avenue Jean Jaurès**
**BP 7073**
**69301 Lyon Cedex 07 (FR)**

(56) Documents cités:
**WO-A1-94/04197** **FR-A1- 3 001 154**
**FR-A1- 3 001 154** **US-A1- 2017 367 982**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 4 065 174 B1

## Description

L'invention concerne un ferrofluide huileux biocompatible comprenant des nanoparticules magnétiques à base d'oxyde de fer dispersées dans une phase huileuse comportant au moins un ester d'acide gras, les nanoparticules magnétiques étant fonctionnalisées en surface par un ou plusieurs phospholipides. L'invention concerne également le procédé de préparation d'un tel ferrofluide huileux biocompatible, et son utilisation en tant qu'agent de contraste ou dans le cadre d'un traitement contre le cancer par hyperthermie déclenchée sous induction magnétique. Enfin, l'invention concerne une nanoémulsion comprenant un tel ferrofluide huileux biocompatible.

Un ferrofluide est constitué d'une dispersion cinétiquement stable de nanoparticules superparamagnétiques dans un liquide porteur pouvant être un solvant aqueux ou organique, ou une huile. Un ferrofluide devient magnétique lors de l'application d'un champ magnétique extérieur statique. Un ferrofluide est susceptible de se déplacer ou de se déformer sous l'action du champ magnétique. Dans le cadre d'une utilisation en hyperthermie déclenchée par induction magnétique, les particules magnétiques sont utilisées comme médiateur d'échauffement sous l'action d'un champ magnétique alternatif externe.

Selon l'utilisation de ces ferrofluides, les nanoparticules magnétiques sont dispersées en milieu aqueux, en milieu huileux ou dans une émulsion.

A l'heure actuelle, les ferrofluides aqueux sont notamment connus pour être utilisés comme agents de contraste en IRM et dans la thérapie de tumeurs solides par hyperthermie magnéto-induite. Par exemple, dans le cadre de la thérapie Nano Therm® développée par la société MagForce AG®, des nanoparticules d'oxyde de fer en suspension aqueuse sont injectées dans la tumeur et s'agglomèrent sur le tissu cible. Les nanoparticules génèrent un dégagement de chaleur sous l'effet d'un champ magnétique externe appliqué, ce qui provoque la destruction des cellules tumorales. Ce traitement peut être appliqué en complément d'une chimiothérapie ou d'une radiothérapie.

Un ferrofluide efficace doit permettre de détruire un grand volume tumoral en des temps de traitement par magnéto-induction courts et pour une concentration en nanoparticules faible. Cependant, les performances calorifiques du milieu aqueux dispersant sont moins bonnes que celles d'un milieu huileux (qu'il soit ou non inclu dans une émulsion). En effet, généralement, la capacité calorifique de l'huile est bien inférieure à celle de l'eau et sa conductivité thermique est également bien inférieure. Aussi, afin d'améliorer l'efficacité de la composition de ferrofluide dans le traitement de l'hyperthermie magnétique, il serait préférable d'utiliser une composition huileuse de ferrofluide, ou au moins une dispersion des nanoparticules magnétiques au sein d'une phase huileuse d'une émulsion.

Par ailleurs, l'injection par voie systémique de ferrofluide aqueux à un patient pose le problème de la quantité de particules magnétiques présentes au niveau de la cible qui doit être suffisante pour compenser les pertes thermiques dans le milieu vivant qui est un environnement aqueux thermorégulé à 37°C. L'amélioration de l'efficacité d'un tel traitement en administration systémique nécessiterait l'injection d'une quantité élevée de nanoparticules injectées, bien au-delà des doses recommandées (de l'ordre de 0,8 mg Fe/kg pour les agents de contraste d'oxyde de fer) associée à une accumulation suffisante au niveau de la zone pathologique apportée par une pharmacocinétique favorable et un ciblage efficace.

Les nanoémulsions de ferrofluide ou des suspensions huileuses de ferrofluide sont principalement connues pour être utilisées comme agents de contraste en IRM et pour le traitement du cancer par hyperthermie magnétique.

Un exemple de ferrofluide en émulsion utilisé comme agent de contraste est rapporté dans la demande FR 3 001 154, qui décrit une nanoémulsion huile-dans-eau comprenant une phase aqueuse, une phase lipidique comprenant une huile, des glycérides d'acides gras saturés en C6-C18 et des particules magnétiques à base d'un composé de fer recouvertes par un ou plusieurs acides gras en C8-C22, et des tensioactifs comprenant au moins un lipide amphiphile et au moins un ligand de ciblage. Une telle nanoémulsion comporte des additifs, tels que les tensioactifs, nécessaires à la stabilisation de la nanoémulsion, pouvant altérer la biocompatibilité de la composition de ferrofluide. Or, une excellente biocompatibilité est essentielle pour une utilisation médicamenteuse.

Dans ce contexte, la Demanderesse a cherché à améliorer la biocompatibilité des compositions de ferrofluide utilisées à des fins médicamenteuses et leur efficacité (notamment les transfert thermiques par diminution de la vitesse de dissipation de la chaleur dans le milieux vivant dans le cadre d'une utilisation en hyperthermie déclenchée sous induction magnétique) pour l'utilisation visée, tout en ayant une stabilité chimique et colloïdale à une température permettant de restituer l'énergie magnétique des nanoparticules en énergie thermique, et de préférence compatible avec un mode d'administration par injection. Les compositions de ferrofluide de l'invention peuvent être envisagées pour différentes applications médicamenteuses, et notamment pour le traitement du cancer par hyperthermie magnétique ou comme agent de contraste.

Afin d'améliorer l'efficacité des ferrofluides, la Demanderesse s'est plus particulièrement intéressée aux ferrofluides huileux, notamment en vue de leur application pour l'hyperthermie magnétique ou comme agent de contraste, ainsi qu'aux émulsions de ferrofluide.

Les nanoparticules d'oxyde de fer étant biocompatibles, la Demanderesse a cherché à améliorer la biocompatibilité du milieu dans lequel ces nanoparticules sont dispersées. En particulier, la Demanderesse a cherché à utiliser

uniquement des additifs et un liquide porteur biocompatibles, et à proscrire tous ceux qui ne sont pas biocompatibles, et pouvant donc induire une toxicité pour le patient.

Dans ce contexte, l'invention propose de nouveaux ferrofluide huileux biocompatible comprenant des nanoparticules magnétiques à base d'oxyde de fer et une phase huileuse comportant au moins un ester d'acide gras, dans lesquels lesdites nanoparticules magnétiques sont fonctionnalisées en surface par des molécules de un ou plusieurs phospholipides. De manière avantageuse, dans les ferrofluides huileux selon l'invention, les nanoparticules magnétiques à base d'oxyde de fer sont dispersées, et de préférence sous la forme d'une dispersion colloïdale, dans la phase huileuse comportant au moins un ester d'acide gras.

En effet, la formation d'agrégats magnétiques peut causer une baisse d'efficacité de l'échauffement des nanoparticules sous induction (i.e. sous l'action d'un champ magnétique alternatif). Aussi, une attention particulière doit être apportée à la dispersion de ces nanoparticules afin d'éviter autant que possible la formation d'agrégats, et notamment d'agrégats de grande taille.

Aussi, la stabilité colloïdale est une condition nécessaire pour améliorer les performances des compositions de ferrofluide, que ce soit pour leur utilisation en hyperthermie magnétique, comme agent de contraste, ou toute autre application thérapeutique. En général, les nanoparticules d'oxyde de fer sont stabilisées cinétiquement d'un point de vue colloïdal dans des compositions huileuses et dans les émulsions à l'aide de tensioactifs ou agents de dispersion, ce qui évite la formation d'agrégats de nanoparticules magnétiques pouvant nuire à leur efficacité d'échauffement du fait des interactions dipolaires magnétiques notamment. Dans les conditions de température d'utilisation, les nanoparticules magnétiques ne doivent pas floculer sous l'action d'un champ magnétique alternatif ou statique appliqué et rester monophasique. Or, les tensioactifs ou les agents de dispersion sont, bien souvent, non ou faiblement biocompatibles.

La Demanderesse a donc cherché les conditions de dispersibilité des nanoparticules à différentes températures pour l'application en hyperthermie sans employer de tensioactif ni d'agent de dispersion (ou tout autre additif non biocompatible). La Demanderesse a alors travaillé sur la chimie de surface des nanoparticules d'oxydes métalliques employées. En effet, la Demanderesse a observé qu'une fonctionnalisation chimique de surface judicieusement choisie, combinée à la présence de certains composants dans la phase huileuse, permet d'améliorer de manière significative la stabilisation colloïdale des compositions huileuses de ferrofluide dans des conditions de température compatibles avec une injection.

Ainsi, l'invention concerne, de manière préférée, un ferrofluide huileux biocompatible comprenant des nanoparticules magnétiques à base d'oxyde de fer et une phase huileuse comportant au moins un ester d'acide gras, lesdites nanoparticules magnétiques à base d'oxyde de fer formant une dispersion colloïdale dans ladite phase huileuse à partir d'une température appartenant à la gamme allant de 20 à 80°C, caractérisé en ce que lesdites nanoparticules magnétiques sont fonctionnalisées en surface par des molécules de un ou plusieurs phospholipides qui ne recouvrent pas totalement la surface des nanoparticules magnétiques à base d'oxyde de fer, et notamment qui assurent un taux de recouvrement de la surface des nanoparticules magnétiques à base d'oxyde de fer tel que le ou les esters d'acide gras présent(s) dans la phase huileuse ont accès à la surface des nanoparticules magnétiques à base d'oxyde de fer.

Le ferrofluide huileux biocompatible selon l'invention présente avantageusement l'une ou l'autre des caractéristiques suivantes, seule ou en combinaison, voire toutes les caractéristiques suivantes :

- le ferrofluide huileux est exempt d'eau et/ou exempt de tensioactif ;
- les molécules de phospholipide(s) assurent un recouvrement de 19 à 76 %, de préférence de 29 à 76 %, et préférentiellement de 34 à 50%, de la surface des nanoparticules magnétiques à base d'oxyde de fer ;
- la densité surfacique de fonctionnalisation (également nommée densité surfacique de greffage) en molécules de phospholipide(s) appartient à la gamme allant de 0,32 molécule/$nm^2$ à 1,22 molécules/$nm^2$, de préférence de 0,48 molécule/$nm^2$ à 1,22 molécules/$nm^2$; et préférentiellement de 0,56 molécule/$nm^2$ à 0,79 molécules/$nm^2$ ;
- le ou les phospholipides contiennent au moins une chaîne grasse, de préférence 2 chaînes grasses, en particulier des chaînes hydrocarbonées, saturées ou mono- ou polyinsaturées, ramifiées ou de préférence linéaires, en C6-C30 et de préférence en C8-C24, voire en C10-C22, et en particulier en C18 ;
- la phase huileuse comprend au moins 70% en masse d'ester(s) d'acide gras, de préférence la phase huileuse comprend de 80% à 95% en masse d'ester(s) d'acide gras, par rapport à la masse totale de la phase huileuse ;
- le ou les esters d'acide gras de la phase huileuse sont choisis parmi les triglycérides d'acide gras saturés en C6-C12, de préférence en C6-C10, les propylènes glycols d'acide gras saturés en C6-C12, de préférence en C6-C10, utilisés seuls ou en mélange ;
- la teneur en nanoparticules magnétiques appartient à la gamme allant de 0,01% à 50% en masse, de préférence de 0,1% à 10% en masse, par rapport à la masse totale de ferrofluide huileux; lorsqu'il est question de teneur en nanoparticules magnétiques, celle-ci ne comprend que les nanoparticules magnétiques à base d'oxyde de fer et n'inclut pas la fonctionnalisation ;
- les nanoparticules magnétiques sont sous forme de sphéroïde, de polyèdre tels que les nanocubes, les bipyramides ou les nanoétoiles, de plaquette, de nanobâtonnet, de nanodisque, ou de nanofleur;

- le ou les phospholipides présente(nt) une tête polaire -O(O)P(OH)$O^-$ et, est(sont), de préférence, choisi(s) parmi les sels de l'acide 1,2-dioléoyl-sn-glycéro-3-phosphatidique et de l'acide 1,2-distéaroyl-sn-glycéro-3-phosphatidique;
- le ferrofluide huileux biocompatible comprend en outre un principe actif lipophile, en particulier choisi parmi les anticancéreux, tels que le Paclitaxel, le Docetaxel, ou la Carmustine.

Les nanoparticules magnétiques à base d'oxyde de fer fonctionnalisées, telles que définies dans le cadre de l'invention, quelle que soit sa variante de réalisation, font également partie intégrante de l'invention.

L'invention concerne également un procédé de préparation d'un ferrofluide huileux biocompatible selon l'invention.

Le procédé de préparation d'un ferrofluide huileux biocompatible selon l'invention permet la dispersion des nanoparticules magnétiques sans emploi d'additif pouvant nuire à la biocompatibilité du ferrofluide huileux. Ainsi, avantageusement, le procédé exclut toute utilisation de composés pouvant induire une toxicité pour le patient autre que l'activité médicamenteuse liée à la présence d'un agent anticancéreux ou du traitement par induction visant à détruire les cellules cancéreuses. De préférence, les fluides et solvants utilisés sont biocompatibles, et aucun additif non biocompatible n'est utilisé. Avantageusement, aucun tensioactif ou agent de dispersion n'est employé dans le procédé de préparation d'un ferrofluide huileux selon l'invention.

Afin d'obtenir un ferrofluide huileux ne comportant pas de tensioactif ni d'agent de dispersion et qui comprend des nanoparticules magnétiques sous la forme d'une suspension colloïdale à une température compatible avec une injection, les inventeurs ont développé un procédé particulier comprenant la formation d'une couche de solvatation des nanoparticules magnétiques.

La Demanderesse a développé un procédé de préparation d'un ferrofluide huileux biocompatible permettant une optimisation et un contrôle du taux de fonctionnalisation des nanoparticules magnétiques par des molécules de phospholipide(s). En effet, la Demanderesse a observé lors de ses recherches que le choix des molécules de phospholipide(s) et le taux de fonctionnalisation avaient un impact sur la stabilité et sur l'efficacité du ferrofluide huileux biocompatible.

Ainsi, le procédé de préparation d'un ferrofluide huileux biocompatible selon l'invention comprend les étapes successives suivantes :

a- disposer d'une dispersion aqueuse de nanoparticules magnétiques à base d'oxyde de fer, dans un solvant aqueux qui peut être de l'eau ou un mélange eau/solvant(s) miscible(s) à l'eau,

b - éliminer le solvant aqueux de la dispersion aqueuse de nanoparticules magnétiques,

c - obtenir un sol colloïdal de nanoparticules magnétiques par ajout de solvant ou mélange de solvants organiques volatils S2,

d - fonctionnaliser en surface lesdites nanoparticules magnétiques du sol colloïdal par des molécules d'au moins un phospholipide,

e - éliminer ledit ou lesdits solvants organiques volatils S2 et disperser les nanoparticules magnétiques fonctionnalisées dans une phase huileuse comportant au moins un ester d'acide gras.

Le procédé de préparation d'un ferrofluide huileux biocompatible selon l'invention comporte avantageusement en outre une étape c2, ultérieure à l'étape c et antérieure à l'étape d, d'ajout d'acide. Cette étape permet d'augmenter l'affinité de la tête polaire des molécules de phospholipide(s) avec la surface des nanoparticules magnétiques en vue d'une fonctionnalisation plus efficace.

L'invention concerne également un médicament, et en particulier un médicament pour le traitement du cancer comprenant un ferrofluide huileux biocompatible selon l'invention, ou obtenu par le procédé de préparation d'un ferrofluide huileux biocompatible selon l'invention.

L'invention concerne encore un ferrofluide huileux biocompatible selon l'invention, ou obtenu selon le procédé de préparation d'un ferrofluide huileux biocompatible selon l'invention, pour son utilisation lors d'un traitement du cancer par hyperthermie déclenchée sous induction magnétique. L'invention concerne aussi une méthode de traitement thérapeutique du cancer par hyperthermie magnétique comprenant l'injection intratumorale d'un ferrofluide huileux biocompatible selon l'invention, ou d'un ferrofluide huileux biocompatible obtenu selon le procédé de préparation selon l'invention, puis l'application d'un champ magnétique alternatif externe.

L'invention concerne aussi une nanoémulsion huile-dans-eau comprenant de 10% à 30% en masse d'un ferrofluide huileux biocompatible selon l'invention, ou d'un ferrofluide huileux biocompatible obtenu selon le procédé de préparation selon l'invention, une phase aqueuse, et au moins un tensioactif. Une telle nanoémulsion peut comprendre en outre des agents de dispersion et/ou des ligands de ciblage. De préférence, la nanoémulsion est biocompatible.

L'invention concerne aussi la préparation d'une nanoémulsion selon l'invention. Ce procédé comprend les étapes successives suivantes :

i - disposer d'un ferrofluide huileux biocompatible selon l'invention, ou obtenu selon le procédé de préparation d'un ferrofluide huileux biocompatible selon l'invention,
ii - disposer d'une phase aqueuse comprenant au moins un tensioactif, et
iii - mélanger la phase aqueuse et le ferrofluide huileux biocompatible de manière à former une nanoémulsion.

La nanoémulsion selon l'invention, ou la nanoémulsion obtenue selon le procédé selon l'invention, peut être utilisée en administration systémique pour le traitement du cancer. Ainsi, l'invention concerne également un médicament, en particulier pour le traitement du cancer comprenant une nanoémulsion selon l'invention, ou obtenue selon le procédé de préparation de nanoémulsion selon l'invention. L'invention concerne encore une nanoémulsion selon l'invention, ou obtenue selon le procédé de préparation de nanoémulsion selon l'invention, pour son utilisation lors du traitement du cancer par hyperthermie magnétique. L'invention concerne aussi une méthode de traitement thérapeutique du cancer par hyperthermie magnétique comprenant l'injection systémique d'une nanoémulsion selon l'invention, ou d'une nanoémulsion obtenue selon le procédé de préparation selon l'invention, puis l'application d'un champ magnétique.

Enfin, l'invention concerne un produit de contraste, en particulier pour l'IRM (Imagerie par Résonnance Magnétique) comprenant un ferrofluide huileux biocompatible selon l'invention, ou une nanoémulsion comprenant le ferrofluide huileux selon l'invention.

Diverses autres caractéristiques ressortent de la description faite ci-dessous en référence aux dessins annexés qui montrent, à titre d'exemples non limitatifs, des formes de réalisation de l'objet de l'invention.

[Fig. 1] La figure 1 est une représentation schématique 1) d'un ferrofluide selon l'invention et 2) d'une nanoémulsion selon l'invention.

[Fig. 2] La figure 2 représente les analyses de spectroscopie infrarouge en réflexion diffuse (DRIFT) de nanoparticules magnétiques non fonctionnalisées et de nanoparticules magnétiques fonctionnalisées avec du DOPA.

[Fig. 3] La figure 3 représente les rendements de chimisorption en DOPA déterminés par mesures ATG pour différentes quantités engagées sur des nanoparticules sphéroïdes FF1 en présence d'acide.

[Fig. 4] La figure 4 représente les thermogrammes de nanoparticules fonctionnalisées avec correspondant à 2000% de la quantité nécessaire pour former une monocouche de DOPS à 7 min et à 30 min de réaction.

[Fig. 5A-C] Les figures 5A, 5B et 5C représentent les profils cinétiques de température des dispersions de nanoparticules sphéroïdes FF1 et de morphologie nanofleurs FF2 et FF3, respectivement, soumises à une induction magnétique (de couple champ fréquence de : (Fig 5A) 755 kHz, 10,2 kA/m, (Fig 5B) 473,5 kHz, 13,36 kA/m et (Fig 5C) 473,5 kHz, 13,36 kA/m) dispersées dans de l'eau ou dans du Miglyol M840® après fonctionnalisation par la DOPA avec des densités de recouvrement théoriques en molécules de phospholipide(s) respectivement de 0,81 et 1,29 et 1,10 molécules/$nm^2$ (température de régulation $T_0$=37°C et $CFe_2O_3$= 5 g/L où $CFe_2O_3$ est la concentration massique en $Fe_2O_3$). En insert de la figure 5A, agrandissement du profil de température sur la plage [0 ;10s].

[Fig. 6A-C] Les figures 6A, 6B et 6C représentent les profils cinétiques de température des dispersions de nanoparticules de morphologie nanofleurs FF2 et FF3 dispersées dans l'eau ou dans du Miglyol M840® après fonctionnalisation par la DOPA avec une densité surfacique de recouvrement nominale en molécules de phospholipide(s) respectivement de 1,29 et 1,10 molécules/$nm^2$, soumises pour chacune des dispersions à deux cycles d'induction magnétique (de couple champ fréquence de : (Fig 6A) dispersion de nanoparticules FF2 à 473,5 kHz, 13,36 kA/m, (Fig 6B) dispersion de nanoparticules FF2 à 344,5 kHz, 16,23 kA/m et (Fig 6C) dispersion de nanoparticules FF3 à 473,5 kHz, 13,36 kA/m, température de régulation $T_0$=37°C et $CFe_2O_3$= 5 g/L).

[Fig. 7A-B] Les figures 7A et 7B représentent les profils cinétiques de température et les valeurs de puissance thermique dissipée par les nanoparticules (représentée par la valeur de SAR, débit d'absorption spécifique ou en anglais « spécifie absorption rate ») des dispersions de nanoparticules de morphologie nanofleurs FF3 en fonction du volume du milieu de dispersion : (Fig 7A) dans de l'eau ou (Fig 7B) dans du Miglyol M840® après fonctionnalisation par la DOPA pour une densité surfacique de recouvrement nominale en molécules de phospholipide(s) de 1,10 molécules/$nm^2$, soumises pour chacune des dispersions à une induction magnétique de couple champ fréquence de 473,5 kHz, 13,36 kA/m, une température de régulation $T_0$=37°C et une concentration massique en oxyde de fer $CFe_2O_3$= 5 g/L.

[Fig. 7C] La figure 7C représente les profils cinétiques de température correspondant à 1 µL de dispersion de nanoparticules FF3 à 5 g/L dans l'eau et dans le Miglyol M840®, déposée directement au bout de la sonde de

température sous induction magnétique (nommée AMF : 473,5 kHz ; 13,36 kA/m, $T_0$=25°C, température ambiante de la pièce).

[Fig. 8] La figure 8 représente le profil cinétique de température des nanoparticules FF3 dispersées dans le Miglyol M840®, après fonctionnalisation par la DOPA pour une densité surfacique de recouvrement nominale en molécules de phospholipide(s) 1,10 molécules/$nm^2$, de concentration massique en oxyde de fer $Fe_2O_3$ de 300 g/L et pour un volume de 1 μL déposé au bout de la sonde de température ($T_0$=25°C, température ambiante de la pièce).

[Fig. 9] La figure 9 représente les profils cinétiques de température de nanoémulsions (1) et (4) ($CFe_2O_3$=12 g/L) contenant les nanoparticules sphéroïdes FF1 dispersées dans le Miglyol M840 émulsifié en phase aqueuse, sous induction à 473,5 KHz, 13,36 kA/m (température de régulation $T_0$=37°C) pendant 20 secondes.

[Fig. 10A-B] La figure 10A montre les images de bioluminescence réalisées suite à l'injection intratumorale du ferrofluide huileux tel que décrit à l'exemple 6, dans une tumeur sous-cutanée d'une souris (deux injections de 1 μL de ferrofluide huileux à 300 g/L sur le même site d'injection) avant (T0) et 24h après (T24) le traitement sous induction (473,5 KHz, 13,36 kA/m). La figure 10B montre les images de fluorescence (FRI, fluorescence réflectance imaging) et de bioluminescence (BLI) de la tumeur *ex vivo* plus de 24h après traitement sous induction.

[Fig. 11A-B] La figure 11A montre les images de bioluminescence réalisées suite à l'injection intratumorale du ferrofluide huileux tel que décrit à l'exemple 6, dans une tumeur sous-cutanée d'une souris (trois injections de 1 μL de ferrofluide huileux à 300 g/L à différents endroits de la tumeur) avant (T0) et 24h après (T24) le traitement sous induction (473,5 KHz, 13,36 kA/m). La figure 11B montre les images de fluorescence (FRI) et de bioluminescence (BLI) de la tumeur *ex vivo* plus de 24h après traitement sous induction.

## Ferrofluide huileux biocompatible

L'invention concerne un ferrofluide huileux biocompatible comprenant des nanoparticules magnétiques fonctionnalisées en suspension dans une phase huileuse comportant au moins un ester d'acide gras, tel que schématisé à la figure 1.

Les nanoparticules magnétiques fonctionnalisées utilisées dans le cadre de l'invention sont des nanoparticules à base d'oxyde de fer qui sont fonctionnalisées en surface par des molécules d'au moins un phospholipide. L'oxyde de fer a l'avantage d'être biocompatible. Avantageusement, les nanoparticules utilisées dans le cadre de l'invention ne comportent pas d'élément métallique pouvant être toxique, tels que du cobalt ou du manganèse.

Dans le cadre de l'invention, « nanoparticules » fait référence à des particules de taille élémentaire nanométrique, c'est-à-dire de taille moyenne élémentaire supérieure à 1 nm et inférieure à 100 nm présentant, de préférence, une distribution de taille monomodale d'écart-type inférieur à 30% en nombre par rapport à la valeur moyenne. Par taille élémentaire, on entend la plus grande dimension de la nanoparticule. Dans le cadre de l'invention, la taille élémentaire d'une nanoparticule magnétique fonctionnalisée correspond à la dimension de la nanoparticule à base d'oxyde de fer en tant que telle et ne comprend pas la fonctionnalisation avec les molécules de phospholipide(s).

De préférence dans le cadre de l'invention, les nanoparticules magnétiques ont une taille moyenne élémentaire inférieure à 30 nm, de préférence inférieure à 25 nm. De préférence, les nanoparticules magnétiques utilisées dans le cadre de l'invention ont une taille moyenne élémentaire supérieure à 3 nm, de préférence supérieure à 5 nm. De préférence, les nanoparticules magnétiques fonctionnalisées ont une taille moyenne élémentaire appartenant à la gamme allant de 5 à 34 nm, de préférence de 7 à 24 nm.

Par « taille moyenne élémentaire », on entend la taille moyenne des nanoparticules inorganiques sans le revêtement de phospholipides et non agrégées. La taille moyenne élémentaire correspond à la moyenne arithmétique des tailles élémentaires mesurées sur un ensemble de nanoparticules, en particulier sur un ensemble de 200 nanoparticules. La taille élémentaire des nanoparticules peut être mesurée par microscopie électronique en transmission (MET), après élimination de la phase huileuse.

Dans les ferrofluides huileux selon l'invention, les nanoparticules magnétiques à base d'oxyde de fer fonctionnalisées conformément à l'invention forment une dispersion colloïdale dans la phase huileuse utilisée, à partir d'une température appartenant à la gamme allant de 20 à 80°C, de préférence dès une température égale à 60 ou 70°C, préférentiellement, dès une température égale à 37°C, et de manière encore plus préférée dès une température égale à 20 ou 25°C. De manière classique, on entend par dispersion colloïdale, des particules solides dispersées dans une phase liquide stable, pendant au moins 24 heures, c'est-à-dire qui ne sédimentent pas et ne s'agrègent pas. La stabilité colloïdale des dispersions de ferrofluides magnétiques en fonction de la température du milieu peuvent être caractérisées qualitativement par leur limpidité et leur translucidité, par opposition à une suspension d'agrégats ou de floculats qui présente un caractère turbide, trouble ou opaque. Une évaluation quantitative peut être réalisée par des mesures de

transmittance à 800 nm. A titre d'exemple, des dispersions de nanoparticules de concentration massique de 0,2 g/L en $Fe_2O_3$-γ dans la gamme de taille allant de 5 à 15 nm est qualifiée d'instable lorsque de telles dispersions ont perdu au moins 30% de leur transmittance à 800 nm par rapport à celle d'une dispersion stable de même concentration. De telles mesures de transmittance peuvent être réalisées avec un spectrophotomètre VARIAN Cary 500 équipé d'un régulateur de température.

La stabilité colloïdale en fonction du taux de recouvrement de surface en molécules de phospholipides peut également être qualifiée par mesure de diffusion dynamique de la lumière (DLS) - qui permet d'obtenir la taille hydrodynamique des nanoparticules en prenant en compte la sphère de solvatation des nanoparticules. Ainsi pour des nanoparticules de 10 nm, des diamètres hydrodynamiques de 40 nm à 50 nm peuvent être mesurés pour des conditions optimales, autour de 100 nm pour des conditions en limite de domaine de stabilité et très supérieures à 100 nm lorsque les dispersions ne sont pas stables.

D'autres techniques sont utilisées pour obtenir des informations sur les nanoparticules comme la diffraction des rayons X (DRX) qui permet d'obtenir la taille du domaine de cohérence cristallin selon des protocoles décrits dans les exemples.

Les nanoparticules utilisées dans le cadre de l'invention peuvent être de formes variées, telles que sous forme de sphéroïde, de polyèdre tel que les nanocubes, les bipyramides ou les nanoétoiles, de plaquette, de nanobâtonnet, de nanodisque, ou de nanofleur. Les nanoparticules dites nanofleurs possèdent une morphologie typique en forme de fleur résultant de l'assemblage de nanocristallites épitaxiées formant de multiples bourgeonnements.

Dans le cadre de l'invention, « sphéroïde » signifie sphérique ou quasi sphérique, c'est-à-dire qui présente un indice de sphéricité (i.e. le rapport entre son plus grand diamètre et son plus petit diamètre) inférieur à 1,2.

Selon un premier mode de réalisation préféré de l'invention, les nanoparticules magnétiques fonctionnalisées sont sous forme sphéroïde, et en particulier sphérique ou quasi-sphérique. Selon ce mode de réalisation, les nanoparticules magnétiques ont alors une taille moyenne élémentaire appartenant à la gamme allant de 5 à 20 nm, de préférence de 7 à 15 nm.

Selon un deuxième mode de réalisation préféré de l'invention, les nanoparticules magnétiques fonctionnalisées utilisées dans le cadre de l'invention sont sous forme de nanofleur, de préférence monocristalline ou quasimonocristalline. Selon ce mode de réalisation, les nanoparticules magnétiques ont alors une taille moyenne élémentaire appartenant à la gamme allant de 10 à 34 nm, de préférence de 10 à 24 nm.

Dans le cadre de l'invention, les nanoparticules employées sont des nanoparticules à base d'oxyde de fer fonctionnalisées en surface par des molécules d'au moins un phospholipide. Selon un mode de réalisation particulier, les nanoparticules magnétiques sont des nanoparticules uniquement composées d'oxyde de fer dont la surface est fonctionnalisée par des molécules de phospholipide(s).

Dans le cadre de l'invention, on entend par nanoparticules « à base d'oxyde de fer » des nanoparticules essentiellement constituées d'oxyde de fer, voire constituées exclusivement d'oxyde de fer.

Les nanoparticules d'oxyde de fer peuvent être des nanoparticules ferrimagnétiques, et typiquement des particules de magnétite ($Fe_3O_4$) ou de maghémite (γ-$Fe_2O_3$) ou tout autre sesquioxyde cubique de formule $[Fe^{3+}]_{Td}[Fe^{3+}{}_{1+2z/3}Fe^{2+}{}_{1-z}V_{z/3}]_{Oh}O_4$ (Td et Oh représentant respectivement les sites tétraédriques et octaédriques de la spinelle et V les lacunes cationiques) dans laquelle z varie de 0 à 1. De préférence, les nanoparticules d'oxyde de fer sont des nanoparticules de maghémite, c'est-à-dire un sesquioxyde cubique de formule $[Fe^{3+}]_{Td}[Fe^{3+}{}_{1+2z/3}Fe^{2+}{}_{1-z}V_{z/3}]_{Oh}O_4$ avec z=1.

Dans le cadre de l'invention, les nanoparticules magnétiques du ferrofluide huileux biocompatible sont fonctionnalisées en surface par un ou plusieurs phospholipides, c'est-à-dire que des molécules de phospholipide(s) identiques ou différents sont liées par liaison chimique à la surface de chaque nanoparticule magnétique. Ces liaisons chimiques sont de préférence des liaisons de coordination ou complexation établies avec la tête polaire des molécules phospholipidiques et un site de fer de surface. En particulier, la tête phospholipidique, notamment du type $-O(O)P(OH)O^-$, est liée par une ou plusieurs liaisons de coordination aux nanoparticules à base d'oxyde de fer.

Par « phospholipide », on entend, de manière classique, une molécule composée d'une tête polaire de type phosphate et d'une ou plusieurs chaînes grasses. De préférence, les molécules de phospholipide sont liées via leur tête polaire à la surface de la nanoparticule.

De préférence, les nanoparticules magnétiques du ferrofluide huileux biocompatible sont fonctionnalisées en surface par un seul type de phospholipide. Bien que non préféré, il peut être envisagé de fonctionnaliser les nanoparticules magnétiques par deux phospholipides différents, ou plus.

Dans le cadre de l'invention, les molécules de phospholipide(s) contiennent au moins une chaîne grasse, de préférence au moins deux chaînes grasses, et en particulier deux chaînes grasses. Ces chaînes grasses sont typiquement des chaînes hydrocarbonées, saturées ou insaturées, linéaires ou ramifiées, et comprenant de 6 à 30 atomes de carbone. Ces chaînes grasses peuvent être différentes ou, de préférence, identiques pour chaque phospholipide utilisé.

De préférence, la ou les chaînes grasses des molécules de phospholipide(s) sont linéaires. Sans vouloir être lié par une quelconque théorie, la Demanderesse est d'avis que ces chaînes grasses des molécules de phospholipide(s)

s'intercalent avec les chaînes grasses des esters d'acide gras de la phase huileuse, permettant ainsi la création d'une sphère de solvatation. L'absence de ramification sur les chaînes grasses des molécules de phospholipide(s) facilite cette intercalation.

Les chaînes grasses des molécules de phospholipide(s) peuvent être saturées, ou mono- ou polyinsaturées. Avantageusement, les chaînes grasses des molécules de phospholipide(s) sont monoinsaturées.

Les chaînes grasses des molécules de phospholipide(s) comportent avantageusement de 6 à 30 atomes de carbone, de préférence de 8 à 24 atomes de carbone, de préférence de 10 à 22 atomes de carbone, et en particulier 18 atomes de carbone.

Dans le cadre de l'invention, la fonctionnalisation des nanoparticules magnétiques a lieu, de préférence, via la tête polaire des molécules de phospholipide(s) : une liaison chimique entre des sites d'oxydes de fer présents en surface des nanoparticules magnétiques et la tête polaire des molécules de phospholipide(s) permet un ancrage fort et une fonctionnalisation solide. Ainsi, cette fonctionnalisation de surface permet de conférer une bonne stabilité chimique et n'est pas détériorée dans les conditions physiologiques et de stockage.

La tête polaire des molécules de phospholipide(s) utilisés pour fonctionnaliser en surface les nanoparticules magnétiques du ferrofluide huileux selon l'invention sont des fragments phosphates et ont de préférence un encombrement stérique faible. La Demanderesse a en effet constaté lors de ses recherches qu'un faible encombrement stérique permettait de mieux contrôler la cinétique et le taux de fonctionnalisation. A titre d'exemples de tête polaire des molécules de phospholipide(s) pouvant être liée à la surface des nanoparticules magnétiques selon l'invention, on peut citer les fragments phosphates issus de l'acide phosphorique, de la phosphatidylethanolamine, de la phosphatidylethanol, du phosphothioethanol ou leurs sels. De manière particulièrement préférée, la tête polaire est le groupe $-O(O)P(OH)O^-$.

Les molécules de phospholipide(s) peuvent être avantageusement choisies parmi les formes salines de glycérophospholipides, comme par exemple l'acide 1,2-dioléoyl-sn-glycéro-3-phosphatidique (DOPA), la 1,2-dioléoyl-sn-glycéro-3-phosphatidyl-L-sérine, la 1,2-dioléoyl-sn-glycéro-3-phosphatidylethanolamine, l'acide 1,2-distéaroyl-sn-glycéro-3-phosphatidique (DSPA), la phosphatidylinositol, la 1,2-dipalmitoyl-sn-glycéro-3-phosphothioethanol, la 1,2-dioleoyl-sn-glycero-3-phosphoethanolamine-N-[3-(2-pyridyldithio)propionate], ou issus des sphingolipides comme la sphingomyéline. Les DOPA et DSPA sous forme anionique sont particulièrement préférées.

De manière préférée, les molécules de phospholipide(s) assurent un recouvrement de 19 à 76 %, de préférence de 29 à 76 %, et préférentiellement de 34 à 50 %, de la surface des nanoparticules magnétiques à base d'oxyde de fer.

La densité surfacique de fonctionnalisation des nanoparticules magnétiques par des molécules de phospholipide(s) appartient de préférence à la gamme allant de 0,32 molécule/$nm^2$ à 1,22 molécules/$nm^2$, de préférence de 0,48 molécule/$nm^2$ à 1,22 molécules/$nm^2$, et préférentiellement de 0,56 molécule/$nm^2$ à 0,79 molécules/$nm^2$. La densité de fonctionnalisation peut être déterminée, comme dans les exemples, grâce à une analyse thermogravimétrique (ATG).

Selon un mode de réalisation, les nanoparticules sont de morphologie sphéroïde et la densité surfacique de fonctionnalisation de ces nanoparticules par des molécules de phospholipide(s) appartient à la gamme allant de 0,32 molécule/$nm^2$ à 1,22 molécules/$nm^2$, de préférence de 0,48 molécule/$nm^2$ à 1,22 molécules/$nm^2$, et préférentiellement de 0,56 molécule/$nm^2$ à 0,79 molécules/$nm^2$.

Selon un mode de réalisation particulièrement préféré, les ferrofluides selon l'invention présentent les caractéristiques suivantes :

- les nanoparticules magnétiques sont de morphologie sphéroïde, de préférence avec une taille moyenne élémentaire appartenant à la gamme allant de 5 à 20 nm, de préférence de 7 à 15 nm,
- la tête polaire des molécules de phospholipide est le groupe $-O(O)P(OH)O^-$ ; et, de préférence, les nanoparticules sont fonctionnalisées avec l'anion phosphate de l'acide 1,2-dioléoyl-sn-glycéro-3-phosphatidique, ou de l'acide 1,2-distéaroyl-sn-glycéro-3-phosphatidique,
- la densité surfacique de recouvrement des nanoparticules magnétiques par des molécules de phospholipide(s) appartient à la gamme allant de 0,32 molécule/$nm^2$ à 1,22 molécules/$nm^2$, de préférence de 0,48 molécule/$nm^2$ à 1,22 molécules/$nm^2$, et préférentiellement de 0,56 molécule/$nm^2$ à 0,79 molécules/$nm^2$,
- l'ester d'acide gras est choisi parmi les triglycérides des acides caproïque, caprique et caprylique et les esters de propylènes glycol d'acide caproïque, caprique et caprylique, et leurs mélanges,
- la phase huileuse comprend au moins 70% en masse d'ester(s) d'acide gras, de préférence la phase huileuse comprend de 80% à 95% en masse d'ester(s) d'acide gras, par rapport à la masse totale de la phase huileuse,
- le ou les esters d'acide gras de la phase huileuse sont choisis parmi les triglycérides d'acide gras saturés en C6-C12, de préférence en C6-C10, les propylènes glycols d'acide gras saturés en C6-C12, de préférence en C6-C10, utilisés seuls ou en mélange.

Selon un autre mode de réalisation, les nanoparticules sont de morphologie nanofleur.

Selon un premier mode de réalisation, les nanoparticules magnétiques fonctionnalisées présentes dans le ferrofluide huileux biocompatible selon l'invention sont des nanoparticules de maghémite fonctionnalisées en surface

par des molécules d'un phospholipide ayant une tête polaire phosphatidylsérine et au moins une, et de préférence deux, chaînes grasses linéaires, insaturées et de préférence monoinsaturées, en C10-C22, et notamment en C18. De préférence selon ce mode de réalisation, les nanoparticules de maghémite sont fonctionnalisées en surface par la 1,2-dioléoyle-sn-glycéro-3-phosphatidyl-L-sérine. Selon ce mode de réalisation, la densité surfacique de recouvrement effective en molécules de phospholipide(s) sur chaque nanoparticule appartient, par exemple, à la gamme allant de 0,48 à 1,22 molécules/$nm^2$ et de préférence de 0,56 à 0,79 molécules/$nm^2$.

Selon un deuxième mode de réalisation préféré, les nanoparticules magnétiques présentes dans le ferrofluide huileux biocompatible selon l'invention sont des nanoparticules de maghémite fonctionnalisées en surface par un phospholipide ayant une tête polaire -O(O)P(OH)O et au moins une, et de préférence deux, chaînes grasses linéaires, insaturées et de préférence monoinsaturées, en C10-C22, et notamment en C18. De préférence selon ce mode de réalisation, les nanoparticules de maghémite sont fonctionnalisées en surface par la forme saline de l'acide 1,2-dioléoyl-sn-glycéro-3-phosphatidique. Selon ce mode de réalisation, la densité surfacique de recouvrement des molécules de phospholipide(s) sur chaque nanoparticule appartient avantageusement à la gamme allant de 0,32 molécule/$nm^2$ à 1,22 molécules/$nm^2$, de préférence de 0,48 molécule/$nm^2$ à 1,22 molécules/$nm^2$, et préférentiellement de 0,56 molécule/$nm^2$ à 0,79 molécules/$nm^2$.

De préférence, la teneur en nanoparticules magnétiques (celle-ci ne comprend que les nanoparticules magnétiques à base d'oxyde de fer et n'inclut pas la fonctionnalisation) dans le ferrofluide huileux appartient à la gamme allant de 0,01 à 50% en masse par rapport à la masse totale de ferrofluide huileux, de préférence de 0,1 à 10% en masse. En d'autres termes, la teneur massique en nanoparticules magnétiques par litre de ferrofluide huileux biocompatible appartient avantageusement à la gamme allant de 0,01 g/L à 500 g/ L, de préférence de 1 g/L à 100 g/L.

Dans le cadre de l'invention, les nanoparticules magnétiques fonctionnalisées sont dispersées ou mises en suspension dans une phase huileuse, et forment une dispersion colloïdale. Cette phase huileuse est avantageusement exempte d'eau, c'est-à-dire qu'elle comporte moins de 0,1% en masse d'eau, de préférence moins de 0,05 % en masse d'eau, et mieux encore moins de 0,01% en masse d'eau par rapport à la masse totale de phase huileuse.

La phase huileuse comprend au moins un ester d'acide gras, et de préférence au moins 70% en masse d'ester(s) d'acide gras, de préférence de 80% à 95% en masse d'ester(s) d'acide gras, par rapport à la masse totale de la phase huileuse.

Dans le cadre de l'invention, les esters d'acide gras sont des esters d'acide carboxylique à chaîne aliphatique comprenant de 4 à 36 atomes de carbone.

Les chaînes grasses des esters d'acide gras peuvent être soit mono- ou polyinsaturées, soit saturées. Un mélange d'ester d'acides gras saturés et insaturés peut également être utilisé dans le cadre de l'invention.

Les chaînes grasses des esters d'acides gras peuvent être ramifiées ou, de préférence, linéaires. Sans vouloir être lié par une quelconque théorie, la Demanderesse est d'avis que ces chaînes grasses des esters d'acide gras s'intercalent avec les chaînes grasses des molécules de phospholipide(s)des nanoparticules fonctionnalisées, permettant ainsi la création d'une sphère de solvatation. L'absence de ramification sur les chaînes grasses des esters d'acides gras facilite cette intercalation.

De préférence dans le cadre de l'invention, les esters d'acide gras sont choisis parmi les esters d'acide gras saturés en C6-C18, de préférence en C6-C12, et en particulier en C6-C10. De préférence, les esters d'acide gras sont choisis parmi les esters d'acide gras saturés en C6-C12, et en particulier en C6-C10. A titre d'exemples, l'acide gras de l'ester d'acide gras peut être choisi parmi l'acide caproïque, l'acide caprylique, l'acide caprique, l'acide laurique, l'acide palmitique, l'acide stéarique, l'acide oléique, l'acide linoléique, l'acide linolénique et l'acide docosahexaénoïque.

De préférence dans le cadre de l'invention, les esters d'acide gras sont choisis parmi les triglycérides d'acide gras saturés en C6-C18, de préférence en C6-C12, et en particulier en C6-C10, et les propylènes glycols d'acide gras saturés en C6-C12, et en particulier en C6-C10, utilisés seuls ou en mélange. De préférence, les esters d'acide gras sont choisis parmi les triglycérides d'acide gras saturés en C6-C12, et en particulier en C6-C10, et les propylènes glycols d'acide gras saturés en C6-C12, et en particulier en C6-C10, utilisés seuls ou en mélange.

A titre d'exemples d'esters d'acide gras pouvant être utilisés dans le cadre de l'invention, on peut citer les triglycérides d'acides gras saturés tel que les triglycérides des acides caproïque, caprique ou caprylique, ou les propylènes glycols d'acides gras saturés tel que le propylène glycol dicaprylocaprate, utilisés seuls ou en mélange.

Selon un premier mode de réalisation, la phase huileuse comprend un seul ester d'acide gras choisi parmi les triglycérides d'acides gras en C6-C10 et les propylène glycols d'acides gras en C6-C10. Selon ce mode de réalisation, l'ester d'acide gras est avantageusement choisi parmi les triglycérides des acides caproïque, caprique et caprylique et les esters de propylènes glycol d'acide caproïque, caprique et caprylique.

Selon un second mode de réalisation, la phase huileuse comprend un mélange d'esters d'acides gras, en particulier 2 ou 3 esters d'acides gras. Selon ce mode de réalisation, les esters d'acide gras sont avantageusement choisis parmi ceux d'acide caprylique, d'acide caprique et d'acide laurique.

La phase huileuse peut comporter en outre une ou plusieurs huiles distinctes dudit ester d'acide gras, et éventuellement un ou plusieurs additifs lipophiles. Avantageusement, ces huiles et additifs lipophiles additionnels sont

biocompatibles et ne génèrent aucune toxicité pour le patient lorsque le ferrofluide huileux selon l'invention lui est administré. Selon un mode de réalisation particulier, le ferrofluide huileux selon l'invention ne comprend pas de tensioactif ni d'agent de dispersion.

Toute huile biocompatible peut être utilisée dans le cadre de l'invention.

A titre d'exemples, la phase huileuse du ferrofluide huileux biocompatible peut comprendre une huile choisie parmi l'huile de soja, l'huile d'olive, l'huile de sésame, l'huile de coton, l'huile d'oeillette, l'huile de coprah, l'huile de noix de coco, l'huile de graines de lin, l'huile de tournesol, l'huile de triglycérides d'acides gras saturés en C6-C12, et les huiles de poissons.

Selon un mode de réalisation préféré de l'invention, la phase huileuse comprend au moins un ester d'acide gras et au moins une huile, et de préférence une seule huile distincte de l'ester d'acide gras. Selon ce mode de réalisation, l'ester d'acide gras est de préférence choisi parmi les triglycérides des acides caprique ou caprylique, le propylène glycol dicaprylocaprate, utilisés seuls ou en mélange.

De préférence, la phase huileuse du ferrofluide huileux biocompatible selon l'invention comprend moins de 30 % en masse d'huile(s) autre que l'au moins un ester d'acide gras par rapport à la masse totale de phase huileuse, et de préférence de 20 à 5 % en masse.

Le ferrofluide huileux biocompatible peut comprendre en outre d'autres additifs lipophiles biocompatibles. A titre d'exemples d'additifs lipophiles biocompatibles, on peut citer les principes actifs lipophiles, tels que les principes actifs lipophiles anticancéreux comme le Paclitaxel, le Docetaxel, ou la Carmustine. D'autres additifs lipophiles biocompatibles, connus dans l'état de l'art, peuvent être utilisés dans le cadre de l'invention et ne seront pas détaillés ici.

De préférence, le pourcentage massique de nanoparticules magnétiques dans la phase huileuse appartient à la gamme allant de 0,01 % à 50 %, de préférence de 0,1% à 10 % par rapport à la masse totale de ferrofluide huileux.

Avantageusement, le ferrofluide huileux biocompatible selon l'invention est stable : les nanoparticules magnétiques sont bien dispersées, ne floculent pas à partir d'une température appartenant à la gamme allant de 20 à 80°C et en particulier à partir de 37°C et encore mieux à partr de 25, voire 20°C. Les dispersions colloïdales sont obtenues à pression atmosphérique (1013, 25 hPa). Lorsque l'on dit que lesdites nanoparticules magnétiques à base d'oxyde de fer fonctionnalisées forment une dispersion colloïdale dans ladite phase huileuse à partir d'une température appartenant à la gamme allant de 20 à 80°C, cela ne signifie pas que la dispersion colloïdale est obtenue sur toute cette gamme de température. Cela signifie que la stabilité est obtenue au moins à une température de la gamme, et en particulier sur au moins une partie de la gamme, notamment dans la gamme haute de température pour laquelle toutes les nanoparticules décrites se dispersent et restituent toute leur chaleur sous induction magnétique. Cependant, il est préférable que les nanoparticules magnétiques à base d'oxyde de fer fonctionnalisées forment une dispersion colloïdale dans ladite phase huileuse à température ambiante, et notamment lorsque la température appartient à la gamme 20-25°C. Avantageusement, la stabilité ces dispersions colloïdales est conservée pendant au moins 24 heures, de préférence pendant au moins 1 mois après obtention. Le caractère colloïdal de la dispersion et sa stabilité peuvent être vérifiés, sous air ambiant et à pression atmosphérique (1013,25 hPa) notamment, sur une durée de 24 heures.

Dans le cadre de l'invention, le domaine de température dans lequel le ferrofluide huileux biocompatible est stable, est dépendant de la densité surfacique de fonctionnalisation en molécules de phospholipide(s), de leur nature chimique et de la taille et morphologie des nanoparticules magnétiques.

## Procédé de préparation d'un ferrofluide huileux biocompatible selon l'invention

L'invention concerne également le procédé de préparation d'un ferrofluide huileux biocompatible selon l'invention tel que détaillé ci-dessus. Ce procédé comprend les étapes successives suivantes :

a- disposer d'une dispersion aqueuse de nanoparticules magnétiques à base d'oxyde de fer, dans un solvant aqueux qui peut être de l'eau ou un mélange eau/solvant(s) miscible(s) à l'eau,

b - éliminer le solvant aqueux de la dispersion aqueuse de nanoparticules magnétiques,

c - obtenir un sol colloïdal de nanoparticules magnétiques par ajout de solvant ou mélange de solvants organiques volatils **S2,**

d - fonctionnaliser en surface lesdites nanoparticules magnétiques du sol colloïdal par des molécules d'au moins un phospholipide,

e - éliminer ledit ou lesdits solvants organiques volatils **S2** et disperser les nanoparticules magnétiques fonctionnalisées dans une phase huileuse comportant au moins un ester d'acide gras.

A l'étape a, les nanoparticules magnétiques ne sont pas fonctionnalisées par les molécules de phospholipide(s) telles que décrites ci-dessus pour les nanoparticules fonctionnalisées du ferrofluide huileux biocompatible selon l'invention. Ces nanoparticules, après une étape de fonctionnalisation en surface (étape d), correspondent aux nanoparticules fonctionnalisées du ferrofluide huileux selon l'invention. Ces nanoparticules non fonctionnalisées comprennent donc

des oxydes de fer, de préférence sont uniquement composées d'oxyde de fer, et en particulier choisi parmi les oxydes de fer décrits pour les nanoparticules magnétiques fonctionnalisées du ferrofluide huileux biocompatible selon l'invention. En milieu aqueux, lesdits oxydes de fer peuvent être hydrolysés en surface de la nanoparticule générant des sites hydroxyle de fer en surface. Lorsqu'elles sont dispersées en phase aqueuse, ces nanoparticules d'oxyde de fer présentent alors à leur surface des charges électrostatiques positives ou négatives selon les valeurs de pH du milieu. Par ailleurs, ces nanoparticules non fonctionnalisées ont la même forme que les nanoparticules fonctionnalisées du ferrofluide huileux biocompatible selon l'invention.

Ces nanoparticules magnétiques non fonctionnalisées sont en suspension en milieu aqueux. Le milieu aqueux peut notamment être composé d'eau éventuellement en mélange avec un ou plusieurs solvant(s) miscible(s) à l'eau tel que le méthanol, l'éthanol, l'isopropanol, le tétrahydrofurane, le diméthylsulfoxide, le diméthylformamide, l'acétonitrile, l'acétone, ou l'acétate d'éthyle. De préférence, le milieu aqueux est uniquement composé d'eau et peut éventuellement comprendre des contre-ions spectateurs issus de la stabilisation du ferrofluide en milieu acide (comme le nitrate ou le perchlorate et leur forme acide à pH 2,5) ou en milieu basique (comme le potassium, le tétraméthylammonium et leur base correspondante à pH 10).

La concentration en nanoparticules magnétiques dans la dispersion aqueuse n'est pas limitante. Avantageusement, elle est supérieure à 1g/L, de préférence, supérieure à 10 g/L, voire supérieure à 100 g/L.

De préférence, la dispersion aqueuse de nanoparticules non fonctionnalisées est uniquement constituée d'un solvant aqueux et de nanoparticules non fonctionnalisées. Bien que non préféré, la dispersion aqueuse de nanoparticules non fonctionnalisées peut également comporter des additifs hydrophiles, tels que des agents stabilisants stériques d'origine macromoléculaire comme la poly(vinylpyrrolidonne), le dextrane, des poly(oxyde d'éthylène) fonctionnalisés ou des tensioactifs comme le β-octyl glucoside, le tween® 80, le dodécylsulfate de sodium.

La dispersion aqueuse de nanoparticules magnétiques non fonctionnalisées à base d'oxyde de fer peut être préparée selon toute technique connue de l'homme de l'art.

L'étape b du procédé de préparation du ferrofluide huileux biocompatible selon l'invention consiste à éliminer le solvant aqueux de la dispersion aqueuse de nanoparticules magnétiques non fonctionnalisées. En d'autres termes, cette étape consiste à éliminer l'eau utilisée en tant que solvant (ou tout autre solvant miscible à l'eau éventuellement présent dans le milieu aqueux) dans la dispersion aqueuse de nanoparticules non fonctionnalisées, ainsi que l'eau physisorbée permettant alors d'obtenir des agrégats de nanoparticules magnétiques non fonctionnalisées, sans solvant.

Pour cela, différentes techniques connues dans l'état de l'art peuvent être employées :

- utilisation d'un aimant afin de séparer les nanoparticules métallique du milieu aqueux, le milieu étant ôté par aspiration ou par pipetage par exemple,
- extraction sur colonne magnétique,
- ultrafiltration,
- centrifugation,
- lavages successifs avec un solvant organique miscible à l'eau tel que le méthanol, l'éthanol, l'isopropanol, le tétrahydrofurane, le diméthylsulfoxide, le diméthylformamide, l'acétonitrile, l'acétone, ou l'acétate d'éthyle,
- lyophilisation,
- pervaporation.

De préférence, l'élimination de l'eau et du ou des solvant(s) miscible(s) à l'eau éventuellement présent(s) est réalisée à la suite de la floculation des nanoparticules. La floculation est obtenue lors de la neutralisation du milieu ou par addition d'un solvant miscible à l'eau tel que l'acétone ou l'éthanol. Le milieu aqueux comprenant l'eau est ensuite séparé des nanoparticules floculées : on emploie, par exemple, un aimant afin de retenir les agrégats de nanoparticules magnétiques non fonctionnalisées, tandis que le milieu est aspiré ou pompé à l'aide d'une pompe à membrane, par exemple.

Ensuite, quelle que soit la méthode d'élimination du solvant aqueux employée, des lavages successifs avec un solvant organique polaire volatil et miscible à l'eau (solvant nommé S1 par la suite), tel que l'éthanol, peuvent permettre de supprimer toute l'eau physisorbée des nanoparticules.

Avantageusement, cette étape d'élimination d'eau permet de récupérer les nanoparticules magnétiques non fonctionnalisées avec une teneur en eau inférieure à 0,25%, de préférence inférieure à 0,025 % en masse par rapport à la masse de nanoparticules magnétiques non fonctionnalisées selon leur surface développée.

L'étape c du procédé de préparation consiste à préparer un sol colloïdal de nanoparticules magnétiques non fonctionnalisées.

Dans le cadre de l'invention, on entend par « sol colloïdal » une dispersion colloïdale homogène de particules solides dans un milieu continu liquide.

Le sol colloïdal de nanoparticules non fonctionnalisées peut être réalisé en dispersant les nanoparticules non fonctionnalisées dans un milieu dispersif.

La mise en dispersion peut être réalisée par toute technique connue de l'homme de l'art, telle que l'agitation par vortex et homogénéisation au bain à ultrasons.

De préférence, le milieu dispersif permet également de solubiliser le ou les phospholipides utilisés ultérieurement pour fonctionnaliser en surface les nanoparticules magnétiques. Aussi, le milieu dispersif comprend, voire est exclusivement constitué de, un ou plusieurs solvants organiques volatils (nommés solvants **S2**). En effet, de tels solvants organiques volatils permettent non seulement la mise en dispersion des nanoparticules non fonctionnalisées, mais également la solubilisation des phospholipides qui seront utilisés lors de l'étape d de fonctionnalisation : ainsi, la cinétique, le rendement de la réaction de fonctionnalisation de surface et le contrôle du taux de greffage en phospholipides s'en trouvent grandement améliorés.

De manière préférée, le milieu dispersif utilisé, et en particulier le ou les solvants organiques volatils **S2,** est biocompatible. Ainsi, si des traces de ce milieu dispersif sont toujours présentes dans le ferrofluide huileux selon l'invention, aucune toxicité pour le patient ne sera générée.

De préférence, le milieu dispersif utilisé est volatil afin de faciliter son élimination par évaporation sous pression réduite lors de l'étape e du procédé. Ainsi, pour le cas où le milieu dispersif n'est pas biocompatible, aucune toxicité n'est due à ce dernier.

A titre d'exemples de milieu dispersif de type solvant organique volatil **S2,** on peut citer le chloroforme seul ou en mélange avec du méthanol (en un ratio 2 :1 v/v par exemple), l'éther diéthylique, l'heptane, le dichlorométhane, l'isopropanol.

Selon un mode de réalisation particulier, le milieu dispersif est constitué d'un seul solvant organique volatil **S2,** de préférence polaire. Selon ce mode de réalisation, le milieu dispersif est avantageusement du chloroforme.

De préférence, la concentration en nanoparticules magnétiques dans le sol colloïdal appartient à la gamme allant de 0,1 à 50 % en masse par rapport à la masse totale de sol colloïdal, de préférence de 1 à 10 % en masse. De telles concentrations en nanoparticules magnétiques permettent avantageusement un bon rendement de production et un contrôle du taux de greffage en termes de distribution des molécules de phospholipide(s) à la surface des nanoparticules magnétiques suite à l'étape de fonctionnalisation.

Une étape optionnelle c2, réalisée après l'étape c et avant l'étape d, peut être réalisée, notamment afin de favoriser la complexation de la tête polaire phosphatidique des molécules de phospholipide(s) avec les sites de fer présents en surface des nanoparticules magnétiques, en vue de l'étape de fonctionnalisation des nanoparticules (étape d). Pour ce faire, un acide organique est ajouté au sol colloïdal de nanoparticules magnétiques non fonctionnalisées. De préférence, un acide organique faible est utilisé. Par acide faible, on entend un acide dont la réaction de dissociation dans l'eau n'est pas totale. La cinétique de chimisorption des molécules de phospholipide(s) s'en trouve améliorée.

Avantageusement, l'acide organique utilisé pour cette étape est biocompatible.

De préférence, l'acide organique utilisé pour cette étape optionnelle c2 est choisi parmi les acides organiques faibles miscibles dans le solvant organique volatil **S2** utilisé à l'étape c pour obtenir le sol colloïdal, et avantageusement à moins de 10% en volume.

A titre d'exemples d'acides organiques pouvant être utilisés pour activer les nanoparticules magnétiques non fonctionnalisées par des molécules de phospholipide(s), on peut citer l'acide acétique, l'acide lactique, l'acide propanoïque, et/ou l'acide butanoïque.

L'étape d du procédé de préparation du ferrofluide huileux biocompatible consiste à fonctionnaliser en surface les nanoparticules magnétiques du sol colloïdal par au moins un phospholipide. Pour cela, le sol colloïdal est mis en présence du ou des phospholipides. Par exemple, une solution de phospholipide(s) peut être ajoutée au sol colloïdal sous agitation mécanique et à pression ambiante. Toute technique connue de formation de sol colloïdal peut être utilisée.

La solution de phospholipide(s) utilisée peut comprendre un ou plusieurs phospholipides, selon la fonctionnalisation des nanoparticules magnétiques recherchée, et au moins un solvant dans lequel le ou les phospholipides sont solubles, nommé ci-après solvant S3. De manière avantageuse, le(s) phospholipide(s) portent une fonction phosphate, sous forme salifiée, notamment avec un cation alcalin tel qu'un atome de sodium, pour favoriser la coordination/complexation de la fonction phosphate de la tête polaire dudit phospholipide, sur les nanoparticules magnétiques.

Les phospholipides sont généralement sous forme de sels pour des questions de stabilité.

Le solvant **S3** peut être un solvant organique. De préférence, ce solvant **S3** est volatil.

A titre d'exemples de solvants **S3** pouvant convenir, on peut citer le chloroforme, seul ou en mélange avec du méthanol (par exemple dans un ratio 2 :1 v :v), l'éther diéthylique, l'heptane, le dichlorométhane, l'isopropanol.

De préférence, ce solvant (ou mélange de solvants) **S3** est le même que le solvant organique volatil **S2** utilisé en tant que milieu dispersif dans le sol colloïdal de nanoparticules magnétiques.

Les phospholipides employés dans cette solution de phospholipides sont ceux détaillés pour les molécules de phospholipide(s) fonctionnalisant les nanoparticules magnétiques du ferrofluide huileux biocompatible selon l'invention.

La teneur en phospholipides dans la dispersion de nanoparticules magnétiques dans les solvants organiques **S2** et **S3** dépend de la surface totale développée par les nanoparticules magnétiques utilisées, du phospholipide utilisé et sa surface développée, des esters d'acide gras utilisés. Avantageusement, la teneur en phospholipides dans le milieu

réactionnel est comprise entre 0,005 et 10% en masse, de préférence de 0,05 à 2,5% en masse par rapport à la masse totale de sol colloïdal.

Selon un premier mode de réalisation, les molécules de phospholipide(s) sont présentes en excès par rapport à la surface développée par les nanoparticules magnétiques non fonctionnalisées, de préférence correspondant à 1000% de la quantité nécessaire pour former une monocouche saturée de phospholipides. En effet, un excès de phospholipides permet de déplacer l'équilibre réactionnel et d'augmenter la cinétique de chimisorption des phospholipides à la surface des nanoparticules magnétiques. Un tel excès est avantageux lorsque cette réaction de fonctionnalisation n'est pas cinétiquement favorable, par exemple lorsque la tête polaire de phosphate des molécules de phospholipide(s) utilisé(s) est encombrée stériquement. Le temps de réaction des molécules de phospholipide(s) contenues dans le sol colloïdal est alors le paramètre à considérer selon l'excès de molécules de phospholipide(s). L'arrêt de la réaction est réalisé par addition d'un solvant entrainant la floculation des nanoparticules et la précipitation de l'excédent de molécules de phospholipide(s), comme l'éthanol par exemple. Ainsi, par exemple, pour un excès de 2000% en phospholipides à tête polaire encombrée telle que la phosphatidylsérine, le temps de chimisorption nécessaire à la dispersion des nanoparticules magnétiques dans les huiles décrites dans le cadre de l'invention est choisi dans l'intervalle de 5 à 30 minutes, de préférence de 6 à 10 minutes, et encore mieux de 7 à 8 minutes. Selon ce mode de réalisation, le procédé comprend une étape ultérieure d'élimination de l'excédent de molécules de phospholipide(s), par exemple par lavages successifs, de préférence avec un mélange de solvants organiques, tel qu'un mélange éthanol/chloroforme (3:1), ainsi qu'une ultime étape d'élimination du chloroforme par de l'éthanol.

Selon un second mode de réalisation, la quantité de molécules de phospholipide(s) utilisée est présente en défaut par rapport à la surface développée des nanoparticules magnétiques non fonctionnalisées, de préférence en un pourcentage allant 38% à 100% de la quantité nécessaire pour former une monocouche saturée de phospholipide(s), et en particulier allant de 38% à moins de 100%. En effet, un défaut de molécules de phospholipide(s) permet de contrôler le taux de fonctionnalisation des nanoparticules magnétiques par les molécules de phospholipide(s). Un tel défaut est avantageux lorsque la réaction de fonctionnalisation est cinétiquement très favorable, par exemple lorsque le phospholipide utilisé présente une tête polaire $-O(O)P(OH)O^{-},M^{+}$, avec M qui est un atome de métal alcalin, notamment un atome de sodium

A la fin de l'étape de fonctionnalisation d, le pourcentage de recouvrement et/ou la densité surfacique de fonctionnalisation des nanoparticules magnétiques est tel que décrit pour les nanoparticules magnétiques fonctionnalisées du ferrofluide huileux biocompatible selon l'invention. Les conditions du procédé selon l'invention, sont adaptées par l'homme du métier pour avoir de tels pourcentages de recouvrement et/ou de telles densités surfaciques de fonctionnalisation.

Une fois la fonctionnalisation des nanoparticules magnétiques achevée, le milieu dispersif du sol colloïdal et la quasi-totalité du solvant **S3** de la solution de phospholipide(s) employée sont éliminés (étape e), par exemple par évaporation sous flux de gaz inerte (azote ou argon) pour prévenir de l'oxydation des phospholipides, afin d'obtenir des agrégats de nanoparticules magnétiques fonctionnalisées par des molécules de phospholipides.

Enfin, ces nanoparticules fonctionnalisées sont mises en dispersion dans une phase huileuse comprenant au moins un ester d'acide gras (étape e). Les éventuelles traces de solvants **S2** et **S3** et principalement d'éthanol sont évaporées sous pression réduite, par exemple à l'évaporateur rotatif à 80°C pendant 30 min effectives. La phase huileuse et l'ester d'acide gras sont tels que décrits pour le ferrofluide huileux biocompatible selon l'invention. Cette dispersion sous forme colloïdale dans la phase huileuse se produit soit spontanément à température ambiante, soit avec un chauffage à une température allant de 30 à 80°C le plus souvent, cette température étant déterminée selon le taux de greffage, les molécules de phospholipide(s), l'huile et l'ester d'acide gras employés. Un tel chauffage peut intervenir lors de l'étape e) ou d'un chauffage ultérieur pour obtenir la dispersion colloïdale.

## Utilisation d'un ferrofluide huileux biocompatible selon l'invention

L'invention concerne également l'utilisation d'un ferrofluide huileux biocompatible selon l'invention, ou obtenu selon le procédé de préparation selon l'invention.

Le ferrofluide huileux biocompatible selon l'invention, ou obtenu selon le procédé de l'invention, peut être utilisé en tant que médicament, en particulier pour le traitement du cancer, notamment pour le traitement de nodules ou tumeurs solides bénignes ou malignes, par hyperthermie magnéto-induite.

Avantageusement, ce médicament pour traiter le cancer est constitué, selon un premier mode de réalisation, uniquement du ferrofluide huileux biocompatible. Alternativement, selon un second mode de réalisation non préféré, le médicament comprend le ferrofluide huileux biocompatible ainsi que des agents anticancéreux lipophiles.

Afin de traiter un patient atteint de cancer, une administration intratumorale de ferrofluide huileux biocompatible est réalisée à l'aide de toute technique connue de l'homme de l'art, puis un champ magnétique alternatif externe est appliqué afin de provoquer un échauffement des nanoparticules magnétiques comprises dans le ferrofluide huileux biocompatible. Cet échauffement localisé se dissipe dans tout le volume de la phase huileuse du ferrofluide huileux,

permettant alors la destruction des cellules tumorales.

Selon un mode de réalisation particulier, cet échauffement est accompagné de la libération d'un agent thérapeutique contenu dans un support thermosensible, ou peut être utilisé pour l'activation d'un agent thérapeutique thermoactivable ou induire l'expression génique d'un gène sous contrôle transcriptionel d'un promoteur thermosensible ou exercer un effet synergique avec un autre agent thérapeutique co-administré indépendamment dans le cadre de protocoles de chimiothérapie et/ou de radiothérapie.

Typiquement, le champ magnétique alternatif appliqué se situe dans la gamme allant de 5 à 25 kA/m, tandis que la fréquence se situe dans la gamme allant de 100 à 750 kHz. La durée du couple champ/fréquence appliqué dépend de la dose thermique par unité de volume dissipée pour un ferrofluide huileux de concentration massique en oxyde de fer donné en fonction du volume tumoral à traiter. Ces protocoles de traitement par hyperthermie sont calibrés pour différents couples champ-fréquence selon le type de traitement recherché qui requiert des doses thermiques différentes, comme la thermoablation, la libération d'un agent thérapeutique, l'activation d'un agent thérapeutique thermoactivable ou l'induction de l'expression génique d'un gène sous contrôle transcriptionel d'un promoteur thermosensible.

Selon un premier mode de réalisation, le ferrofluide huileux biocompatible est utilisé en tant que seul médicament dans ce traitement.

Selon un second mode de réalisation, notamment lorsque le ferrofluide huileux ne comprend pas un médicament de chimiothérapie, le ferrofluide huileux biocompatible est utilisé en combinaison avec un autre médicament de chimiothérapie, tels que le Paclitaxel, le Docetaxel, ou la Carmustine, de préférence contenu dans l'huile magnétique. Une co-injection de formulation injectable contenant des principes actifs thermosensibles comme le Thermodox® peut également être envisagée.

Le ferrofluide huileux biocompatible selon l'invention, ou obtenu selon le procédé selon l'invention, peut également être utilisé en tant que produit de contraste pour l'imagerie médicale, telle que l'IRM, l'imagerie optique de fluorescence dans le proche infrarouge (PIR), et les approches locales fibrées par endoscopie.

Dans le cas d'une utilisation en imagerie de fluorescence, le ferrofluide huileux administré au patient comprend de préférence des fluorophores lipophiles.

L'invention concerne aussi une méthode d'imagerie du corps entier ou d'une partie du corps d'un individu comprenant une étape d'obtention d'une ou plusieurs images dudit corps entier ou partie du corps entier par une technique d'imagerie médicale, ledit corps entier ou ladite partie du corps comprenant le produit le contraste comprenant le ferrofluide huileux biocompatible.

Avantageusement, les ferrofluides huileux selon l'invention sont dispersés sous forme colloïdale, au moins à la température qui est atteinte pendant le traitement par induction : les nanoparticules magnétiques sont alors bien dispersées, ne forment pas d'agrégat et ne floculent pas dans le temps.

Encore plus avantageusement, les ferrofluides sont stables en particulier pendant au moins 24 heures, avantageusement pendant au moins 1 mois, à une température appartenant à la gamme 20-40°C à pressbn atmosphérique et, notamment à température ambiante (20 °C) ou physiobgique (37 °C). Il est possible de différencier la stabilité à court terme (durée inférieure ou égale à 24h) qui sera évaluée sous air ambiant et la stabilité à long terme (durée supérieure à 24h). La stabilité à long terme pourra notamment être évaluée en conservant le ferrofluide sous atmosphère inerte tel que l'argon ou l'azote et à l'abri de la lumière et à la température à laquelle la stabilité doit être évaluée, notamment à 20°C ou 37°C.

Dans le cas où les ferrofluides selon l'invention correspondraient à une dispersion colloïdale, uniquement à une température supérieure à la température ambiante, notamment à une température appartenant à la gamme allant de 20 à 40°C, il pourrait être nécessaire de conserver lesferrofluides dans des récipients ou chambres thermostatées ou bien de les chauffer avant utilisation à une température permettant d'obtenir une dispersion colloïdale. Leur administration par injection pourra également se faire par utilisation de perfusions thermostatées ou par mise en oeuvre de kits ou dispositifs chauffants pour maintenir le ferrofluide à une température dans la gamme allant de 20 à 40° C à laquelle la stabilité colloïdale est satisfaisante.

## Nanoémulsion comprenant un ferrofluide huileux biocompatible selon l'invention

L'invention concerne également une nanoémulsion de ferrofluide huileux biocompatible selon l'invention, ou obtenu selon le procédé de préparation d'un ferrofluide huileux selon l'invention, tel que schématisé à la figure 1.

Par « nanoémulsion », on entend une émulsion dont les gouttelettes de phase dispersée dans la phase continue sont de dimension nanométrique, c'est-à-dire comprise entre 100 nm et moins de 300 nm.

Dans le cadre de l'invention, la nanoémulsion peut être une émulsion simple de type huile-dans-eau ou eau-dans-huile, ou une émulsion multiple de type eau-dans-huile-dans-eau. De préférence, la nanoémulsion selon l'invention est une nanoémulsion huile-dans-eau.

Selon un premier mode de réalisation, la phase lipophile de la nanoémulsion est constituée uniquement du ferrofluide huileux biocompatible.

Selon un second mode de réalisation, la phase lipophile de la nanoémulsion comprend le ferrofluide huileux biocompatible en combinaison avec un ou plusieurs autres composés lipophiles choisis notamment parmi les huiles, les fluorophores lipophiles, les perfluorocarbones, les principes actifs lipophiles comme par exemple le paclitaxel, le docétaxel, l'étoposide, la carmustine.

Par ailleurs, pour améliorer leur pharmacocinétique, l'interphase des gouttelettes lipidiques magnétiques peuvent contenir un ou plusieurs lipides PEGylés (phospholipides) et/ou ligands de ciblage (anticorps, peptides, aptamères).

Les composés lipophiles hors principes actifs anti-cancéreux pouvant être utilisés dans la nanoémulsion selon l'invention sont de préférence biocompatibles et ne génèrent aucune toxicité.

Toute huile biocompatible connue dans l'état de l'art peut être utilisée. A titre d'exemples d'huiles pouvant être utilisées dans le cadre de l'invention en tant qu'autre composé lipidique de la nanoémulsion, on peut citer l'huile de soja, l'huile d'olive, l'huile de sésame, l'huile de coton, l'huile d'oeillette, l'huile de coprah, l'huile de noix de Coco, l'huile de graines de lin, l'huile de tournesol, les huiles de triglycérides d'acides gras comme le Miglyol 812N® ou le Labrafac® WL 1349, les huiles de propylène glycol d'acides gras comme le Miglyol 840® ou le Labrafac® PG et les huiles de poissons.

Lorsque la nanoémulsion comprend une huile ou un mélange d'huiles en plus du ferrofluide huileux biocompatible, celle(s)-ci est (sont) présente(s) en une teneur appartenant, en général, à la gamme allant de 10 à 35% en masse, de préférence de 20 à 30% en masse par rapport à la masse totale de nanoémulsion.

Parmi les fluorophores lipophiles pouvant être utilisés dans le cadre de l'invention, on peut citer les analogues fluorescents de phospholipides et de sphingomyeline, les cyanines comme le vert d'indocyanine (ICG) et carbocyanines lipophiles.

Lorsque la nanoémulsion comprend des fluorophores lipophiles, ceux-ci sont de préférence présents en une teneur appartenant à la gamme allant de 0,0001 à 0,02‰ en masse, de préférence de 0,001 à 0,01‰ en masse, par rapport à la masse totale de nanoémulsion.

Les ligands de ciblage sont connus pour permettre de reconnaitre la cible biologique par interaction moléculaire, et ainsi améliorer la spécificité du mélange administré. A titre d'exemples de ligands de ciblage, on peut citer des agents à base d'acides aminés comme l'acide folique, de sucres comme le mannose ou le FDG (Fluoro-Désoxy-Glucose couramment utilisé en imagerie PET), de séquences peptidiques comme le peptide RGD cyclique dirigé contre les intégrines ou les TATE (Tyr3)-octreotate de forte affinité pour les récepteurs de type 2 de la somatostatine, des composés synthétiques comme le raclopride agissant comme antagoniste des récepteurs dopaminergiques D2, des anticorps de différents formats comme ceux des camélidés (nanobodies) ou fragments d'anticorps recombinants sélectionnés par des méthodes de criblage phage display comme le scFv dirigé contre le PSMA ou les anti PD1/PDX1 utilisés en immunothérapies, des aptamères sélectionnés par des méthodes de criblage SELEX dirigés contre des récepteurs membranaires de cellules cancéreuses.

La phase aqueuse de la nanoémulsion selon l'invention est typiquement constituée d'eau, éventuellement en combinaison avec un ou plusieurs solvants miscibles à l'eau comme l'éthanol et le propylène glycol. La phase aqueuse de la nanoémulsion peut également comprendre des sels (chlorures de sodium ou de potassium) ou des tampons.

En outre, la nanoémulsion peut comprendre un ou plusieurs tensioactifs, également nommés agents de dispersion, de préférence biocompatibles. Ces tensioactifs et agents de dispersion sont alors présents à l'interface phase aqueuse/phase lipophile de la nanoémulsion.

A titre d'exemples de tensioactifs et agents de dispersion pouvant convenir dans le cadre de l'invention, on peut citer les lécithines d'oeuf ou de soja, les acides biliaires comme le déoxycholate de sodium, l'huile de ricin polyoxyéthylénée, le polysorbate 20, le polysorbate 40, le polysorbate 60, le polysorbate 80, le monolaurate de sorbitane (span ® 20, span ® 40, span ® 60, et/ou span ® 80), les poloxamères, les PEG block co-polymères.

Lorsque ceux-ci sont présents, la teneur en tensioactifs et/ou en agents de dispersion appartient typiquement à la gamme allant de 1 à 5% en masse, de préférence de 1,8 à 3,8% en masse, par rapport à la masse totale de la nanoémulsion.

Avantageusement, les nanoémulsions selon l'invention sont stables à température ambiante (20°C) à pression atmosphérique. En particulier, les nanoémulsions selon l'invention sont stables pendant au moins 24 heures, de préférence pendant au moins 6 mois à température ambiante (20°C) et à pression atmosphérique.

Dans le cadre de l'invention, la stabilité colloïdale de la nanoémulsion est déterminée par évaluation de la répartition granulométrique par diffusion dynamique de la lumière (DLS), comme détaillé dans les exemples.

## Procédé de préparation d'une nanoémulsion selon l'invention

L'invention concerne également un procédé de préparation d'une nanoémulsion selon l'invention.

Un tel procédé de préparation de nanoémulsion comprend les étapes successives suivantes :

i. disposer d'une phase lipophile comprenant un ferrofluide huileux biocompatible selon l'invention, ou obtenu selon le procédé de préparation d'un ferrofluide huileux biocompatible selon l'invention,

ii. disposer d'une phase aqueuse,
iii. mélanger la phase lipophile et la phase aqueuse de manière à former une nanoémulsion.

En particulier, de préférence, le mélange de l'étape iii, sera effectué à une température où le ferrofluide huileux selon l'invention se trouve sous la forme d'une dispersion colloïdale. La phase lipophile comprenant le ferrofluide huileux biocompatible selon l'invention pourra, également, être chauffé à une telle température avant son mélange avec la phase aqueuse.

Le procédé de préparation de nanoémulsion selon l'invention peut comprendre une étape optionnelle i2, ultérieure à l'étape i et antérieure à l'étape iii, de mise en présence du ferrofluide huileux biocompatible avec un ou plusieurs composés choisis parmi les huiles, les fluorophores lipophiles, les ligands de ciblage, les agents de surface lipophiles, les pharmacophores, tels que décrits ci-dessus, afin d'obtenir la phase lipophile de la nanoémulsion. Cette étape optionnelle peut être réalisée par mélange à température et pression ambiantes selon toute technique connue de l'homme de l'art.

A l'étape ii, la phase aqueuse peut comprendre un ou plusieurs tensioactifs, tels que décrits ci-dessus, lorsqu'il(s) est (sont) présent(s).

A l'étape iii, le mélange des phases aqueuse et lipophile est classiquement réalisé à haute vitesse selon toute technique connue de l'homme de l'art. Par exemple, les phases lipophile et aqueuse, éventuellement préalablement chauffées en particulier à une température dans la gamme allant de 60 à 70 °C, sont d'abord mélangées à haute vitesse afin de former une émulsion grossière, puis homogénéisées à l'aide d'un sonicateur ou par homogénéisation à haute pression.

La phase aqueuse, la phase lipophile, et le(s) tensioactif(s) éventuellement présent(s), sont tels que décrits pour la nanoémulsion selon l'invention.

## Utilisation d'une nanoémulsion selon l'invention

L'invention concerne également l'utilisation d'une nanoémulsion selon l'invention, ou d'une nanoémulsion obtenue selon le procédé de l'invention.

Une nanoémulsion selon l'invention peut être utilisée en tant que médicament, en particulier dans le traitement du cancer par hyperthermie magnéto-induite. Pour cela, une administration systémique de la nanoémulsion est effectuée, suivie de l'application d'un champ magnétique alternatif externe. Dans ce cas, le champ magnétique est appliqué au moment où l'accumulation du produit dans la tumeur a atteint son maximum. Cette durée d'attente entre l'injection et l'application du champ magnétique alternatif est estimée par imagerie optique (fibrée ou non), par IRM ou échographie (pour les formulations à base de perfluorocarbones). Pour le cas d'une administration intratumorale, le champ magnétique peut être appliqué à la suite de l'injection. Les caractéristiques de magnéto-induction (valeurs de couple champ/fréquence et durée d'application) pour les nanoémulsions peuvent être adaptées en fonction des doses thermiques recherchées. L'échauffement des gouttelettes d'huile magnétique peut être accompagné de la libération d'un agent thérapeutique ou peut être utilisé pour l'activation d'un agent thérapeutique thermo-activable ou induire l'expression génique d'un gène sous contrôle transcriptionel d'un promoteur thermosensible ou exercer un effet synergique avec un autre agent thérapeutique co-administré indépendamment dans le cadre de protocoles de chimiothérapie et/ou de radiothérapie.

Selon un premier mode de réalisation, la nanoémulsion est utilisée en tant que seul médicament dans ce traitement.

Selon un second mode de réalisation, notamment lorsque la nanoémulsion ne comprend pas un médicament de chimiothérapie, la nanoémulsion est utilisée en combinaison avec un autre médicament de chimiothérapie de nature lipophile, tels que les taxanes (Paclitaxel et Docetaxel) ou la carmustine. Une co-injection de formulation injectable contenant des principes actifs thermosensibles comme le Thermodox® peut également être envisagée.

Les volumes et les concentrations de ferrofluide huileux administrés, ainsi que le nombre d'injection(s) et le temps entre chaque injection le cas échéant, dépendent notamment de la tumeur traitée (localisation, volume) et du patient (âge, condition physique), et sont déterminées par le praticien.

La nanoémulsion selon l'invention, ou obtenue selon le procédé selon l'invention, peut également être utilisée en tant que produit de contraste pour l'imagerie médicale, telles que l'IRM, l'échographie ou l'imagerie optique de fluorescence dans le proche infrarouge (PIR), les approches locales fibrées par endoscopie.

Dans le cas d'une utilisation en imagerie de fluorescence, la nanoémulsion administrée au patient comprend de préférence des fluorophores lipophiles.

L'invention concerne aussi une méthode d'imagerie du corps entier ou d'une partie du corps d'un individu comprenant une étape d'obtention d'une ou plusieurs images dudit corps entier ou partie du corps entier par une technique d'imagerie médicale, ledit corps entier ou ladite partie du corps comprenant le produit le contraste comprenant la nanoémulsion.

**Kits**

L'invention concerne également un kit comprenant un contenant dans lequel est placé le ferrofluide huileux selon l'invention, ou la nanoémulsion selon l'invention. Le kit peut également comprendre les nanoparticules magnétiques fonctionnalisées en surface par des molécules de un ou plusieurs phospholipide(s) telles que décrites selon l'invention, seule dans un récipient, et éventuellement la phase huileuse comportant au moins un ester d'acide gras, destinée à constituer le ferrofluide huileux dans un autre récipient séparé. Le ferrofluide ou la nanoémulsion peut alors être reconstitué à partir du kit, juste avant son utilisation ou administration. Le kit peut également comprendre des conditions d'utilisation en termes de couples champ/fréquence applicables et de dose thermique dissipée correspondante par unité de volume de ferrofluide de concentration donnée. Il convient de noter que la concentration en fer est également certifiée pour des questions d'indication.

**Exemples**

Matériels et méthodes :

A) Phospholipides testés :

Les phospholipides utilisés pour préparer les ferrofluides huileux dans les exemples ci-dessous sont :

- le 1,2-dioleoyl-sn-glycero-3-phosphoethanolamine (DOPE) de formule Chem. 1 suivante :

[Chem. 1]

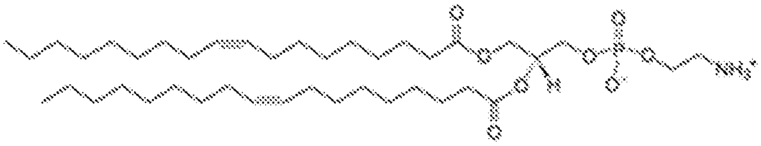

,

- l'acide 1,2-dioléoyl-sn-glycéro-3-phosphatidique (DOPA) de formule Chem. 2 suivante (dans sa forme de sel de sodium) :

[Chem. 2]

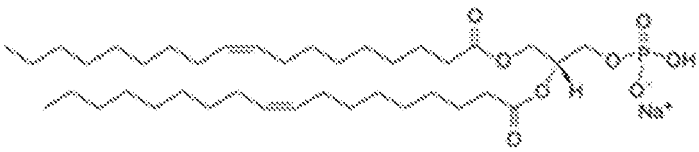

,

- l'acide 1,2-distéaroyl-sn-glycéro-3-phosphatidique (DSPA) de formule Chem. 3 suivante (dans sa forme de sel de sodium):

[Chem. 3.]

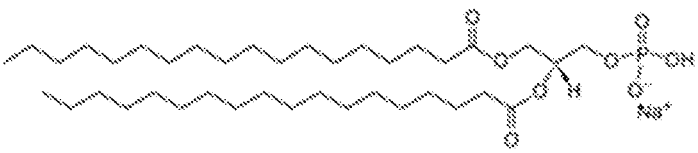

,

et

- le 1,2-dioléoyl-sn-glycéro-3-phospho-L-sérine (DOPS) de formule Chem. 4 suivante (dans sa forme de sel de sodium) :

[Chem. 4]

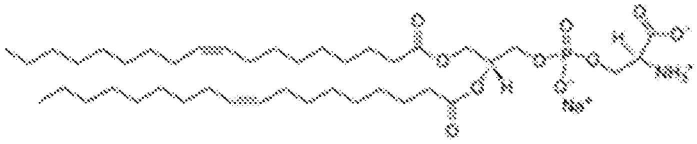

.

B) Composition des phases huileuses :

Les phases huileuses utilisées dans les exemples sont des mélanges de triglycérides d'acides gras ou de propylènes glycols d'acides gras : le Miglyol 812N® et Miglyol 840® respectivement. Le tableau 1 ci-dessous indique les acides gras composant les triglycérides ou les propylènes glycols d'acides gras composant ces huiles (les pourcentages sont des pourcentages massiques par rapport au poids total de l'huile).

[Table 1]

| | Miglyol 812 | Miglyol 840 |
|---|---|---|
| Acide caprylique | 50-56% | 65-80% |
| Acide caprique | 30-45% | 20-35% |
| Acide laurique | 2% | 3% |

C) Dosage du Fer :

Le dosage du fer est réalisé par spectroscopie UV visible en dissolvant des nanoparticules d'oxyde de fer dans une solution d'acide chlorhydrique (HCl) 5M. Après dissolution complète des nanoparticules dans HCl 5M, l'absorbance à 350 nm est mesurée pour déterminer la concentration en fer. La concentration en fer est déterminée selon la formule : $A^{350nm} = \varepsilon.l.[Fe]$. Le coefficient d'extinction molaire $\varepsilon$ vaut 2960 $L^{-1}.mol.cm^{-1}$.

D) Taille des nanoparticules :

D1) Mesure par diffusion dynamique de la lumière (DLS)

Le rayon hydrodynamique des nanoparticules est déterminé par Diffusion Dynamique de la Lumière en utilisant un appareil Cordouan Vasco équipé d'un laser de longueur d'onde de 658 nm et à un angle de 135°, l'acquisition des données se faisant pendant une durée de 60 s.

D2) Mesure par diffraction des rayons X (DRX)

La taille des cristallites est déterminée par DRX en utilisant la formule de Scherrer Math. 1 ci-dessous, où $\Gamma$ est le diamètre moyen des cristallites, $\lambda$ la longueur d'onde des rayons X ($\lambda$(Cu K$\alpha$)=1 ,5406Å), $\beta$ la largeur à mi-hauteur du pic le plus intense et $\Theta$ l'angle de Bragg :

[Math. 1]

$$\Gamma = \frac{0,9\lambda}{\beta * \cos(\Theta)}.$$

D3) Mesure par microscopie électronique à transmission (MET)

La taille élémentaire moyenne des nanoparticules est déterminée par microscopie électronique à transmission en mesurant la taille de 200 nanoparticules à l'aide d'un logiciel de traitement d'images (ImageJ Rasband, W.S., ImageJ, U. S. National Institutes of Health, Bethesda, Maryland, USA, https://imagej.nih.gov/ij/, 1997-2019.), obtenues avec un microscope Philips CM120 opérant à 120 kV et équipé d'une caméra Ultra scan USC1000 (2k × 2k).

E) Caractérisation de surface :

E1) Analyse thermogravimétrique (ATG)

Environ 10 mg de nanoparticules, précédemment séchées sous pression réduite à 70°C, sont mises dans un creuset en plathe, et l'analyse est réalisée en utilisant une thermobalance Tag2400 de chez Setaram. L'échantillon est chauffé jusqu'à 600°C sous air avec une rampe de 5°C/min.

E2) Spectroscopie infrarouge en réflexion diffuse

La spectroscopie infrarouge en réflexion diffuse (DRIFT) est réalisée avec un spectromètre Bruker IFS Equinox 55. Les nanoparticules fonctionnalisées sont séchées à l'étuve à 70°C après les étapes de lavage Puis les nanoparticules sont broyées dans du KBr anhydre (3 % en masse).

E3) Détermination des densités surfaciques de fonctionnalisation et des taux de recouvrement théoriques (également nommés nominaux) et réels

La surface développée par molécule de phospholipide projeté sur une surface dépend du volume occupé par la tête polaire en comparaison avec celui des chaînes lipidiques. Dans le cas des phospholipides le volume d'encombrement des deux parties (polaire et hydrophobe) est équivalent de sorte qu'un lipide s'inscrit dans un cylindre et non dans un cône. C'est cette particularité qui permet de les assembler en membranes vésiculaires et non en micelles. Les valeurs d'encombrement surfacique des phospholipides sont comprises généralement entre 60 et 65 $Å^2$ en fonction de l'environnement dans lequel ils se trouvent, seul ou en mélange, du nombre d'insaturations, des interactions avec une surface solide (membrane lipidique supportée) ou en milieu liquide (vésicule) et de différents paramètres physico-chimiques comme la température. La valeur de référence d'une molécule de phospholipide comprenant une tête phosphatidyl - $O(O)P(OH)O^-$, $Na^+$ ((comme c'est le cas des DOPA et DSPA) est de 62 $Å^2$ correspondant à l'encombrement moléculaire au sein d'une monocouche saturée contenue dans une membrane lipidique. Cette valeur de densité surfacique moléculaire correspond à celle que l'on retrouve pour des membranes lipidiques (bicouches phospholipidiques) en considérant une seule monocouche. Il y a bien sûr de légères différences lorsque la fonction OH est substituée. Ainsi la dioleoylphosphatidylsérine, la surface développée moléculaire est de l'ordre de 65 $Å^2$/molécule et pour la dioleoylphosphatidyléthanolamine elle est généralement mesurée entre 60 et 65 $Å^2$. Elle est donc choisie par la suite à 62 $Å^2$. Cette valeur de 62 $Å^2$ est utilisée dans le cadre de l'invention, pour les calculs des pourcentages de recouvrement de la surface des nanoparticules et pour les calculs des densités surfaciques de fonctionnalisation en molécules de phospholipide, et ce quel que soit le phospholipide envisagé.

On nomme « taux de recouvrement nominal ou théorique » en phospholipide, le pourcentage molaire que représente la quantité de lipide engagée par rapport à la quantité nécessaire pour former une monocouche, calculée à partir de la taille moyenne des nanoparticules mesurée par MET et la surface de référence de la tête polaire du phospholipide engagée. Lorsque les nanoparticules sont des sphéroïdes, il est considéré que la taille moyenne des nanoparticules mesurée par MET est pris comme diamètre pour évaluer la surface moyenne développée par les particules. Pour les nanofleurs, compte tenu de leur rugosité de surface, leur surface développée a été estimée par itérations d'enrobages de silice de différentes épaisseurs par voie sol-gel (voie de synthèse Stôber). Cette méthode a ainsi permis d'estimer la surface de 15% supérieure à une sphère lisse de même diamètre.

Les mesures d'ATG permettent de déterminer la quantité de molécules de phospholipide réellement présente sur les nanoparticules par unité de surface à partir de la perte de masse totale mesurée entre 200°C et 600°C, de la masse molaire des résidus organiques décomposés lors de la combustion complète rapportée à la surface développée par les nanoparticules mises en jeu. Le rendement de greffage représente le rapport entre la quantité de molécules de phospholipide chimisorbées à la surface des nanoparticules et la quantité nominale mise en jeu lors de la réaction.

F) Synthèse des nanoparticules d'oxyde de fer

F1) Synthèse des nanoparticules FF1 par coprécipitation alcaline

Une masse de 31,41 g de $FeCl_2.4H_2O$ (0,158 mol) dissoute dans 170 mL d'acide chlorhydrique 1,5 M, est versée dans un bécher contenant 85,4 g de $FeCl_3.6H_2O$ (0,316 mol) dissout dans 3,5 L d'eau (rapport stoechiométrique initial $Fe^{2+}/Fe^{3+}= 0,5$). Sous forte agitation mécanique, 300 mL d'une solution d'ammoniaque (28-30%, m/m) sont ajoutés rapidement. Le milieu est laissé sous agitation 15 min. Les floculats de nanoparticules de magnétite sont décantés avec l'aide d'un aimant permanent, le surnageant est ensuite éliminé. Après 2 étapes successives de lavage à l'eau, la surface des nanoparticules est oxydée par ajout d'un volume de 200 mL d'$HNO_3$ 2 M puis laissé sous agitation durant 15 min. Après décantation et élimination du surnageant, le coeur des nanoparticules est oxydé en maghémite par ajout de 600 mL d'une solution de nitrate de fer à 0,33 M. Le milieu réactionnel est porté à ébullition durant 30 min. Après décantation et élimination du surnageant, 200 mL d'acide nitrique à 2 M sont ajoutés. Le floculat est ensuite décanté magnétiquement et lavé 3 fois à l'acétone afin d'éliminer l'excès d'acide. Le floculat est enfin peptisé dans 200 mL d'eau. Après évaporation de l'excédent d'acétone, le ferrofluide est ramené à un volume de 1 L avec de l'eau. Par la suite, la dispersion de nanoparticules de maghémite est nommée FF1. A l'issue de la synthèse le ferrofluide présente une concentration massique en oxyde de fer de 69 g/L et développe une surface par unité de volume de 11316 $m^2/L$. La taille élémentaire moyenne des nanoparticules sphéroïdes ainsi obtenues déterminée par MET est de 7,5 nm $\pm$ 2 nm, et de 6,9 nm lorsqu'elle est mesurée par DRX. Le rayon hydrodynamique mesuré est $D_H$ = 38 nm.

F2) Synthèse des nanoparticules FF2 par polyol

Une masse de 1,082 g de $FeCl_2.4H_2O$ (5,44 mmol) et une de 0,398 g de $FeCl_3.6H_2O$ (1,47 mmol) sont dissouts dans 80 g d'un mélange de diéthylène glygol (DEG) et de N-méthyldiéthanolamine (NMDEA) (ratio 1:1, v/v). Cette solution est mélangée 1h sous flux d'azote sous agitation jusqu'à dissolution complète des précurseurs. Une masse de 0,64 g de NaOH (16 mmol) est dissoute dans 40 g d'un mélange NMDEA/DEG (1: 1, v/v) sous flux d'azote. La solution de NaOH est ajoutée à la solution contenant les précurseurs et le mélange est chauffé à 220°C, à 2°C/min, pendant 4 h. Les nanoparticules obtenues sont sédimentées magnétiquement et lavées avec un mélange éthanol / acétate d'éthyle (1:1, v/v) trois fois pour éliminer les impuretés organiques et inorganiques. Un lavage à l'acide nitrique 10% est réalisé. 8,25g de $Fe_3(NO_3)_3.9H_2O$ (20,4 mmol) est solubilisé dans 20 mL d'eau et la solution est ajoutée aux nanoparticules. La dispersion de nanoparticules est chauffée à 80°C pendant 45 minutes pour obtenir de la maghémite. Après décantation et élimination du surnageant, les nanoparticules sont lavées à l'acide nitrique 10%, puis à l'acétone et finalement à l'éther diéthylique. Au final les nanoparticules sont redispersées dans l'eau. Par la suite la dispersion de ces nanoparticules de maghémite est nommée FF2. A l'issue de la synthèse le ferrofluide présente une concentration massique en oxyde de fer de 20 g/L et développe une surface de 1574 $m^2/L$. La taille élémentaire moyenne des nanoparticules de morphologie nanofleurs ainsi obtenues est mesurée à 15,4 nm $\pm$ 3 nm par la méthode MET et à 16 nm par DRX. Le rayon hydrodynamique mesuré est $D_H$ = 26 nm.

Synthèse des nanoparticules FF3 par polyol

La synthèse des nanoparticules FF3 est réalisée dans les mêmes conditions opératoires que celles décrites pour l'obtention des nanoparticules FF2 sauf que la montée de température à 220°C, à 2°C/min, pendant 4 h s'effectue cette fois-ci dans des conditions réactionnelles isolées thermiquement. Pour ce faire, de la laine de verre a été placée autour du ballon en contact des parois du réacteur à l'air libre et au-dessus du chauffe-ballon afin de limiter les déperditions de chaleur, permettant ainsi un meilleur contrôle cinétique de la rampe de température. La taille élémentaire moyenne des nanoparticules de morphologie nanofleurs obtenue par la méthode MET est de 18,5 nm $\pm$ 3,1 nm.

G) Stabilité colloïdale des dispersions huileuses

La stabilité colloïdale des dispersions est observée en fonction de la température en première approximation, visuellement puis par mesure de la transmittance à 800 nm avec un spectrophotomètre VARIAN Cary 500 équipé d'un régulateur de température. La qualité des dispersions des nanoparticules est également vérifiée par DLS (méthode D1). Lorsque dans les tableaux des exemples, la mention « + » figure cela signifie que le caractère colloïdal est obtenu en dispersant les nanoparticules dans la phase huileuse à la température correspondant et que ce caractère colloïdal est toujours présent au moins 24 heures après son obtention, temps s'écoulant au moins entre la préparation de la dispersion et la mesure de la transmittance.

**Exemple 1** : **Dispersion des nanoparticules de FF1, FF2 et FF3 dans du chloroforme**

Cet exemple a pour but de transférer les nanoparticules des ferrofluides aqueux FF1, FF2 et FF3 dans le chloroforme sans recourt à des tensioactifs pour pouvoir réaliser les greffages des phospholipides par chimisorption. Pour ce faire, un volume de 1 mL d'une solution d'ammoniaque (28-30%, m/m) est ajoutée à 35,4 mL de FF1 à 69 g/L en $\gamma$-$Fe_2O_3$. Les floculats de nanoparticules sont décantés à l'aide d'un aimant permanent, le surnageant est ensuite éliminé. Les nanoparticules agrégées sont lavées 2 fois à l'eau. Puis, les nanoparticules agrégées sont lavées 5 fois à l'éthanol. Enfin, un volume de 80 mL de chloroforme est ajouté aux nanoparticules et les nanoparticules sont redispersées au bain à ultrasons pendant 3 minutes. La concentration finale est de 30,5 g/L en $\gamma$-$Fe_2O_3$ dans le chloroforme. Le protocole est identique pour les ferrofluides FF2 et FF3.

**Exemple 2** : **Préparation de ferrofluides huileux biocompatibles issus de la fonctionnalisation, en présence d'acide, de nanoparticules magnétiques par des molécules de phospholipide présentant une tête polaire non encombrée.**

**Exemple 2.1.** : **dispersions de nanoparticules fonctionnalisées avec des molécules de phospholipide mono-insaturé**

**Exemple 2.1.1** : **Dispersion dans le Miglyol M840® de nanoparticules magnétiques du ferrofluide FF1 fonctionnalisées par la DOPA**

Dans cet exemple, une dispersion de nanoparticules magnétiques de FF1 fonctionnalisées par la DOPA dans le Miglyol M840® est préparée. L'encombrement surfacique de référence d'une molécule de phospholipide est de 62 $Å^2$ correspondant à l'encombrement moléculaire au sein d'une monocouche saturée contenue dans une membrane lipidique. Le protocole de fonctionnalisation pour des nanoparticules de taux de recouvrement nominal (correspondant à la quantité de lipide engagée par rapport à la quantité nécessaire pour former une monocouche) de 55 % (soit 0,89 molécule/$nm^2$) par rapport à la quantité nécessaire pour former une monocouche de DOPA est le suivant : Un volume de 16,4 mL de nanoparticules magnétiques ([$\gamma$-$Fe_2O_3$]=30,5 g/L, surface développée=82$m^2$) dispersées dans le chloroforme, préparées dans l'exemple 1, sont diluées dans un volume de 47,7 mL d'une solution d'acide acétique dans le chloroforme (1:9, v/v). À cette dispersion, un volume de 3,51 mL de DOPA (25 mg/mL dans le chloroforme, 45,2 $m^2$) est ajouté sous vortex. Après 14 h de réaction à 4°C, de l'éthanol est ajouté dans le milieu jusqu'à floculation des nanoparticules et l'ensemble est mis à décanter magnétiquement. Le floculat magnétique est lavé avec 3 volumes de 100 mL de mélange chloroforme/éthanol (1:3, v/v) puis avec 2 volumes de 100 mL d'éthanol. A l'issue du dernier lavage, les nanoparticules sont séchées sous flux d'azote et un volume de 15 mL de Miglyol M840® est ajouté aux nanoparticules. Les nanoparticules se dispersent à partir de 35°C dans l'huile après chauffage au bain marie.

De la même manière, une gamme de taux de recouvrement nominaux en DOPA fixée entre 20% et 150% par rapport à la monocouche, correspondant à une densité de greffage théorique comprise entre 0,33 DOPA/$nm^2$ et 2,42 DOPA/$nm^2$, peut être réalisée en travaillant à concentration en DOPA constante (1,25 mg/mL). Pour certaines valeurs nominales de densité, les nanoparticules se dispersent spontanément à température ambiante (20°C).

Le taux de recouvrement nominal en DOPA est calculé selon l'équation Math. 2 suivante.

[Math. 2]

$$\text{taux recouvrement} = 100 * \frac{N^{PL} * 0{,}62}{4\pi * N^{NP} * R^2}$$

où $N^{PL}$ correspond au nombre de molécules de phospholipide, $N^{NP}$ au nombre de nanoparticules et R le rayon de la nanoparticule en nm.

La stabilité des dispersions de nanoparticules issues de FF1 fonctionnalisées par la DOPA dans le Miglyol M840® a été évaluée à différentes températures et différents taux de recouvrement, tel que résumé dans le tableau 3 ci-dessous. Les critères désignant la qualité des dispersions sont : - = non dispersable (milieu turbide), + = sol colloïdal stable dans le temps (dispersion limpide). Ces critères de criblage peuvent être évalués de façon pratique par observations visuelles en parfait accord avec les meures de transmittance à 800 nm. Les mesures d'ATG ont permis de déterminer la quantité de DOPA réelle par unité de surface et le rendement de greffage. Les densités surfaciques de DOPA nominales et réelles ainsi que la stabilité colloïdale des nanoparticules dans le M840® à 20°C et 37°C sont résumées dans le Tableau 2 ci-après.

[Table 2]

| Taux de recouvrement nominal | 20% | 30% | 38% | 44% | 55% | 70% | 90% | 100% | 150% |
|---|---|---|---|---|---|---|---|---|---|
| Taux de recouvrement réel | 19,9% | 28,0% | 29,8% | 34,8% | 35,4% | 36,0% | 45,3% | 49,1% | 75,8% |
| Quantité de DOPA ajoutée par unité de surface $d_{DOPA/nm^2}$ | 0,33 | 0,48 | 0,61 | 0,71 | 0,89 | 1,13 | 1,45 | 1,61 | 2,42 |
| Quantité de DOPA réelle par unité de surface $d_{DOPA/nm^2}$ | 0,32 | 0,45 | 0,48 | 0,56 | 0,57 | 0,58 | 0,73 | 0,79 | 1,22 |
| Stabilité à T=20°C | - | - | - | + | + | + | + | + | - |
| Stabilité à T=37°C | - | - | + | + | + | + | + | + | + |
| Stabilité à T=70°C | + | + | + | + | + | + | + | + | + |
| $D_H$ (nm)* | 489 | 450 | 268 | 112 | 58 | 49 | 42 | 53 | 293 |
| Transmittance à 800 nm (%) à 20°C** | 41 | 44 | 53 | 70 | 75 | 84 | 82 | 83 | 40 |

* $D_H$ : Diamètre hydrodynamique mesuré en DLS à 20°C.

** Les valeurs de transmittance ont été déterminées sur des dispersions à 0,2 g/L en $Fe_2O_3$.

La chimisorption de la DOPA sur les nanoparticules est confirmée par DRIFT (Figure 2) avec la présence d'une bande entre 1070 $cm^{-1}$ et 1170 $cm^{-1}$ et 1200 $cm^{-1}$ correspondant respectivement aux vibrations des liaisons P-OH et P-O. L'analyse ATG permet également de confirmer la chimisorption de la DOPA sur les nanoparticules. Les analyses ATG permettent de déterminer des rendements de greffage réel compris entre 49 % et 97,5% sur toute la gamme, et de 51% à 80% sur le domaine de stabilité colloïdal (Figure 3). Ces rendements élevés sont avantageux car ils permettent une utilisation de molécules de phospholipides minimisée.

**Exemple 2.1.2 : Dispersion dans le Miglyol 812N® de nanoparticules magnétiques du ferrofluide FF1 fonctionnalisées par la DOPA**

Le protocole utilisé est le même que celui décrit dans l'exemple 2.1.1 excepté que les nanoparticules modifiées sont redispersées dans le Miglyol 812N®. La gamme est réalisée dans les même conditions c'est-à-dire à concentration en DOPA constante (1,25 mg/mL).

La stabilité des nanoparticules issues de FF1 fonctionnalisées par la DOPA dans le Miglyol 812N® a été évaluée à différentes températures et différents taux de recouvrement nominaux, tel que résumé dans le tableau 3 ci-dessous. Les critères désignant la qualité des dispersions sont : - = non dispersable (milieu turbide), + = sol colloïdal stable dans le temps (dispersion limpide).

[Table 3]

| Taux de recouvrement nominal | 20% | 25% | 35% | 44% | 50% | 60% | 70% | 80% | 89% | 96% |
|---|---|---|---|---|---|---|---|---|---|---|
| $c_{DOPA}/nm^2$ théorique | 0,33 | 0,40 | 0,56 | 0,71 | 0,81 | 0,96 | 1,12 | 1,29 | 1,44 | 1,55 |
| $c_{DOPA}/nm^2$ réelle | 0,32 | 0,39 | - | 0,56 | 0 57 | 0,59 | 0,60 | 0,67 | 0,73 | 0,79 |
| Dispersibilité à T=20°C | - | - | - | - | + | + | + | - | - | - |
| Dispersibilité à T=35°C | - | - | - | + | + | + | + | + | - | - |
| Dispersibilité à T=70°C | - | - | + | + | + | + | + | + | + | + |

**Exemple 2.1.3** : **Dispersion dans le Miglyol M840® de nanoparticules magnétiques du ferrofluide FF2 fonctionnalisées par la DOPA**

Le protocole de chimisorption de la DOPA est identique à celui décrit dans l'exemple 2.1.1 excepté que les nanoparticules du ferrofluide FF2 sont utilisées.

La stabilité des nanoparticules issues de FF2 fonctionnalisées par la DOPA dans le Miglyol M840® a été évaluée à différentes températures et différents taux de recouvrement nominaux, tel que résumé dans le tableau 4 ci-dessous. Les critères désignant la qualité des dispersions sont : - = non dispersable (milieu turbide), + = sol colloïdal stable dans le temps (dispersion limpide). Les valeurs de diamètre hydrodynamique (DH) des nanoparticules du ferrofluide FF2 ont été mesurées par DLS externalisée (VASCO-FLEX, Cordouan Technologies) pour différents taux de greffage en DOPA, à 60°C sous induction par application d'un champ magnétique alternatif externe (755 kHz, 10,2 $kA.m^{-1}$).

[Table 4]

| Taux de recouvrement nominal | 22% | 31% | 39% | 44% | 53% | 61% | 71% | 79% | 82% |
|---|---|---|---|---|---|---|---|---|---|
| $c_{DOPA}/nm^2$ théorique | 0,34 | 0,48 | 0,60 | 0,69 | 0,82 | 0 ,95 | 1,10 | 1,22 | 1,32 |
| Dispersibilité à T=20°C | - | - | - | - | - | - | - | - | - |
| Dispersibilité à T=35°C | - | - | - | - | - | - | - | - | - |
| Dispersibilité à T=60°C | - | - | - | - | - | + | + | + | + |
| $D_H$ (60°C, en nm) | 342 | 259 | 238 | 322 | 261 | 53 | 39 | 45 | 40 |

**Exemple 2.1.4** : **Dispersion dans le Miglyol M840® de nanoparticules magnétiques du ferrofluide FF3 fonctionnalisées par la DOPA**

Le protocole de chimisorption de la DOPA pour la mise en dispersion des nanoparticules magnétiques dans le Miglyol M840® est identique à celui décrit dans l'exemple 2.1.1 excepté que les nanoparticules du ferrofluide FF3 sont utilisées en appliquant un taux de recouvrement nominal de 1,10 molécules/$nm^2$. Deux dispersions de concentrations massiques égales à 5 g/L et 300g/L sont préparées pour les mesures de SAR (débit d'absorption spécifique ou en anglais, « spécifie absorption rate ») et les expérimentations *in vivo* de thermoablation de tumeurs portées par des souris (exemple 6).

**Exemple 2.2** : **Préparation d'une dispersion de nanoparticules fonctionnalisées avec des molécules de phospholipide présentant une tête polaire substituée** : **Dispersion dans le Miglyol M840® de nanoparticules magnétiques du ferrofluide FF1 fonctionnalisées par la DOPE.**

Le protocole utilisé est le même que celui décrit dans l'exemple 2.1.1, excepté la nature du phospholipide employé. La gamme est réalisée dans les mêmes conditions, c'est-à-dire à concentration en DOPE constante (1,25 mg/mL).

La stabilité des dispersions de nanoparticules issues de FF1 fonctionnalisées par la DOPE dans le Miglyol M840® a été évaluée. Les résultats sont résumés dans le tableau 5 ci-dessous. Ces résultats sont donnés en fonction de la température et du taux de recouvrement. Les critères désignant la qualité des dispersions sont : - = non dispersable (milieu turbide), + = sol colloïdal stable dans le temps (dispersion limpide).

[Table 5]

| Taux de recouvrement nominal | 24% | 48% | 52% | 61% |
|---|---|---|---|---|
| $d_{DOPE/nm2}$ théorique | 0,39 | 0,77 | 0,84 | 0,98 |
| Dispersibilité à T=25°C | - | - | - | - |
| Dispersibilité à T=35°C | - | - | + | - |
| Dispersibilité à T=70°C | - | + | + | + |

A 52% de taux de recouvrement les nanoparticules fonctionnalisées sont dispersables à partir de 35° C. A 70° C la gamme de habilité est élargie entre 48 et 61% de taux de recouvrement nominaux. La présence d'un substituant sur la tête polaire affecte les cinétiques de chimisorption par rapport à celles de la DOPA et réduit la gamme de stabilité colloïdale en composition de surface et température.

**Exemple 2.3.** : **Préparation d'une dispersion de nanoparticules fonctionnalisées avec des molécules de phospholipide saturé à tête polaire non substituée** : **Dispersion dans le Miglyol M840® de nanoparticules magnétiques du ferrofluide FF1 fonctionnalisées par la DSPA.**

Le protocole utilisé est le même que celui décrit dans l'exemple 2.1.1, excepté la nature du phospholipide employé. La gamme est réalisée dans les mêmes conditions, c'est-à-dire à concentration en DSPA constante (1,25 mg/mL).

La stabilité des nanoparticules issues de FF1 fonctionnalisées par la DSPA dans le Miglyol M840® a été évaluée à différentes températures et différents taux de recouvrement, tel que résumé dans le tableau 6 ci-dessous. Les critères désignant la qualité des dispersions sont : - = non dispersable (milieu turbide), + = sol colloïdal stable dans le temps (dispersion limpide).

[Table 6]

| Taux de recouvrement nominal | 10% | 24% | 48% | 58% | 61% |
|---|---|---|---|---|---|
| Dispersibilité à T=25°C | - | - | - | - | - |
| Dispersibilité à T=35°C | - | + | - | - | - |
| Dispersibilité à T=70°C | - | + | + | + | + |
| Température de dispersion | - | 35°C | 45°C | 52°C | 58°C |

La température de dispersion des nanoparticules magnétiques, c'est-à-dire la température à partir de laquelle la dispersion des nanoparticules magnétiques est limpide, dépend de la quantité en DSPA employée. La température de dispersion des nanoparticules magnétiques a été déterminée par des mesures de transmission à 800 nm en température, selon le protocole détaillé ci-dessus.

Les nanoparticules FF1 fonctionnalisées par la DSPA sont dispersables pour des températures supérieures à l'ambiante dans les huiles biocompatibles, à partir de 35 °C à un taux de recouvrement nominal ce 24 %. La température de dispersion dans le Miglyol M840® augmente avec le taux de recouvrement en molécules de phospholipide.

**Exemple 3** : **Préparation de ferrofluides huileux biocompatibles issus de la fonctionnalisation des nanoparticules magnétiques par des molécules de phospholipide en absence d'acide**

**Exemple 3.1.** : **Préparation de ferrofluides huileux biocompatibles par fonctionnalisation de surface des nanoparticules au moyen de molécules de phospholipide présentant des têtes phosphates substituées** : **cas des nanoparticules magnétiques issues de FF1 modifiées avec la DOPS dans le Miglyol M840®**

L'encombrement stérique offert par certaines têtes polaires de phospholipides substituées a pour effet de ralentir la cinétique de chimisorption à la surface de l'oxyde de fer. Néanmoins cette cinétique peut être accélérée en jouant sur l'augmentation de la concentration en phospholipide, c'est-à-dire en introduisant en excès lesdites molécules de phospholipide par rapport à une monocouche de molécules de phospholipide. Dans ce cas, le temps de réaction devient le paramètre clé pour contrôler le nombre de molécules de phospholipide greffées par nanoparticules.

Un volume de 14,2 mL de DOPS (25 mg/mL dans le chloroforme) est ajouté sous vortex à 1,64 mL de nanoparticules magnétiques FF1 ($[Fe_2O_3]$=30,5g/L) dispersées dans le chloroforme. La réaction de chimisorption est stoppée, à différents temps de réaction, en ajoutant de l'éthanol pour inhiber la réaction provoquant la floculation des nanoparticules et la précipitation de l'excédent de molécules de phospholipide. La suspension est ensuite décantée sous aimant. Le floculat est lavé trois fois avec 50 mL d'éthanol. Lors du dernier lavage, les nanoparticules sont séchées sous flux d'azote et un volume de 15 mL de Miglyol M840® est ajouté aux nanoparticules. Les nanoparticules se dispersent spontanément dans l'huile.

La stabilité des nanoparticules issues de FF1 fonctionnalisées par la DOPS dans le Miglyol M840® a été évaluée après différents temps de réaction conduisant à différents taux de recouvrement, tel que résumé dans le tableau 7 ci-dessous. Les critères désignant la qualité des dispersions sont : - = non dispersable (milieu turbide), + = sol colloïdal stable dans le temps (dispersion limpide).

[Table 7]

| Temps incubation (min) | 1 | 7 | 20 | 30 | 60 | 90 |
|---|---|---|---|---|---|---|
| Dispersibilité à T=25°C | - | + | - | - | - | - |

L'ATG permet de suivre la cinétique de chimisorption de la DOPS au cours du temps. Le tableau 8 et la figure 4 montrent les variations de densité réelle de greffage des nanoparticules fonctionnalisées avec 2000% d'excès de DOPS soit après 7 min de réaction, lavage et séchage, soit après 30 min de réaction, lavage et séchage. La figure 4 représente les thermogrammes de nanoparticules fonctionnalisées avec un excès de 20% de DOPS à 7 min et à 30 min de réaction, ainsi obtenus.

[Table 8]

| Temps (min) | ΔM % | $d_{DOPS/nm^2}$ |
|---|---|---|
| 7 min | 19,1 | 0,87 |
| 30 min | 22,5 | 1,02 |

**Exemple 3.2.** : **Préparation de ferrofluides huileux biocompatibles par fonctionnalisation de surface des nanoparticules au moyen de molécules de phospholipide présentant des têtes phosphates non substituées** : **cas des nanoparticules magnétiques issues de FF1 modifiées avec la DOPA dans le Miglyol M840®**

Le protocole de préparation des nanoparticules présentant un défaut en phospholipide de 50% (c'est à dire $d_{DOPA/nm2}$=0,81 DOPA.$nm^{-2}$) est le suivant : un volume de 16,4 mL de nanoparticules magnétiques FF1 ($[\gamma\text{-}Fe_2O_3]$=30,5 g/L) dispersées dans le chloroforme sont diluées dans 44 mL de chloroforme. À cette dispersion, un volume de 3,18 mL de DOPA (25 mg/mL dans le chloroforme) est ajouté sous vortex. Après 14 h de réaction à 4°C, de l'éthanol est ajouté jusqu'à floculation des nanoparticules suivi d'une décantation magnétique. Le floculat est lavé avec un mélange de chloroforme/éthanol (1:3, v/v) puis avec de l'éthanol. Lors du dernier lavage, les nanoparticules sont séchées sous flux d'azote et un volume de 15 mL de Miglyol M840® est ajouté aux nanoparticules. Les nanoparticules se dispersent spontanément dans l'huile.

De la même manière, une gamme de taux de recouvrement nominaux en DOPA située entre 5% et 96% en

défaut par rapport à l'encombrement moléculaire au sein d'une monocouche d'une membrane lipidique, correspondant à une densité de recouvrement comprise entre 0,08 DOPA/nm$^2$ et 1,55 DOPA/nm$^2$, peut être réalisée en travaillant à concentration en DOPA constante (1,25 mg/mL).

La stabilité des nanoparticules issues de FF1 fonctionnalisées par la DOPA dans le Miglyol M840® sans apport d'acide lors de la réaction a été évaluée à différentes températures et différents taux de recouvrement, tel que résumé dans le tableau 9 ci-dessous. Les critères désignant la qualité des dispersions sont : - = non dispersable (milieu turbide), + = sol colloïdal stable dans le temps (dispersion limpide).

[Table 9]

| Taux de recouvrement nominal | 20% | 25% | 35% | 44% | 50% | 60% | 70% | 80% | 89% | 96% |
|---|---|---|---|---|---|---|---|---|---|---|
| $d_{DOPA/nm^2}$ théorique | 0,33 | 0,40 | 0,56 | 0,71 | 0,81 | 0,96 | 1,12 | 1,29 | 1,44 | 1,55 |
| Dispersibilité à T=20°C | - | - | - | - | - | + | + | + | - | - |
| Dispersibilité à T=35°C | - | - | - | + | + | + | + | + | - | - |
| Dispersibilité à T=70°C | - | + | + | + | + | + | + | + | + | + |

**Exemple 4** : **Comparaison de ferrofluides huileux et aqueux issus de FF1, FF2 et FF3**

Des dispersions de nanoparticules de concentration massique en oxyde de fer de 5 g/kg de solvant dans les phases huileuses biocompatibles ou en milieu aqueux sont préparées pour mesurer et comparer leur échauffement sous induction magnétique. La température des échantillons est mesurée par une fibre optique (OTG-M420, OpsenTM) et la température des échantillons est régulée à 37°C.

Les champs magnétiques alternatifs sont générés par deux dispositifs différents, afin d'accéder à différents couples champ/fréquence. Le premier dispositif est le DM3 de chez nB nanoScale Biomagnetics offrant les conditions suivantes : 473,5 kHz et 13,36 kA. m$^{-1}$, 344,5 kHz et 16,23 kA. m$^{-1}$, 217 kHz et 20,09 kA. m$^{-1}$, et 146 kHz, 21,96 kA. m$^{-1}$.

Un deuxième dispositif, constitué d'une bobine à induction alimenté par un générateur Minimax JunioTM 1TS 3,5 kW, permet d'accéder à une condition supplémentaire : 755 kHz, 10,2 kA.m$^{-1}$.

Les mesures de vitesse d'échauffement sous induction magnétique permettent de déterminer la puissance thermique des nanoparticules par unité de masse de liquide magnétique nommée, SAR (Spécifie Adsorption Rate). La SAR est déterminée selon la formule Math. 3 suivante :

[Math. 3]

$$SAR = \frac{m_s}{m_{NP}} C_p \frac{\Delta T}{\Delta t}$$

où $C_P$ représente la capacité calorifique du milieu de dispersion en J.(g.K)$^{-1}$ ($C_{eau}$= 4,18 J.g$^{-1}$.K$^{-1}$ et $C_{huile \approx}$ 2 J.g$^{-1}$.K$^{-1}$ ), $m_S$ la masse de solvant, $m_{NP}$ la masse de nanoparticules et $\Delta T/\Delta t$ représente la pente à l'origine (37°C) de la tangente de la courbe d'évolution de la température en fonction du temps.

Les profils de température des dispersions aqueuses magnétiques FF1, FF2 et FF3 de concentration massique en oxyde de fer égale à 5 g/L, comparés aux profils obtenus avec les nanoparticules obtenues aux exemples 2.1.1, 2.1.3 et 2.1.4, issues respectivement de FF1, FF2 et FF3 fonctionnalisées par la DOPA avec des densités de recouvrement théoriques respectivement de 0,81, 1,29 et 1,10 molécules/nm$^2$ et dispersées dans le Miglyol M840® sous induction magnétique, sont représentés aux figures 5A, 5B et 5C, respectivement pour deux couples champ/fréquence ((Fig 5A) 755 kHz, 10,2 kA/m, (Fig 5B) 473,5 kHz, 13,36 kA/m, (Fig 5C) 473,5 kHz, 13,36 kA/m).

En 10 secondes, la température du ferrofluide aqueux FF1 s'élève de 0,12°C tandis que pour les nanoparticules issues de FF1 fonctionnalisées par la DOPA dispersées dans le Miglyol M840®, l'augmentation est de 0,5°C, correspondant à un gain de température de plus d'un facteur 4. Sur une durée d'induction de 100 secondes, ce même gain est mesuré, soit une élévation de température de plus de 4,8°C dans la phase huileuse et seulement de 1,2°C dans la phase aqueuse. Concernant FF2, en 10 secondes, la température de la dispersion aqueuse FF2 augmente de 0,8°C, tandis que, pour les dispersionsde FF2 fonctionnalisées par la DOPA et dispersées dans le Miglyol M840®, l'augmentation de température est 5,8 fois supérieure soit de 5,2°C. Un gain encore plus élevé est observé pour les dispersions FF3 dans l'huile : en 10 secondes, une augmentation de température de 3 °C est mesurée pour le ferrofluide aqueux, tandis

l'augmentation de température est 12 fois supérieure, soit de 36°C, pour le ferrofluide huileux. La dispersion huileuse de FF3 présente donc des propriétés d'échauffement bien supérieures par rapport à la dispersion aqueuse de FF3, avec une montée en température très rapide.

Les valeurs des SAR mesurées dans les deux milieux (aqueux et huileux) pour les trois types de nanoparticules FF1, FF2 et FF3 sont répertoriées dans le tableau 10 ci-dessous :

[Table 10]

| | Couple champ fréquence appliqué | SAR (W/g) |
|---|---|---|
| FF1 $H_2O$ | 755kHz, 10,2 kA.m$^{-1}$ | 10 |
| FF1 M840 | | 20 |
| FF1 $H_2O$ | 473,5 kHz, 13,36 kA.m$^{-1}$ | 12,1 |
| FF2 $H_20$ | | 83,6 |
| FF2 M840 | | 220 |
| FF3 $H_2O$ | | 295 |
| FF3 M840 | | 2605 |

Les valeurs de SAR à 37°C pour la dispersion FF1 sous induction à 755 kHz, 10,2 kA/m sont doublées entre le milieu aqueux et huileux. Les valeurs de SAR à 37°C pour les deux types de nanoparticules magnétiques de morphologie nanofleur issues des dispersions FF2 et FF3 soumise à une induction à 473,5 kHz, 13,36 kA/m sont multipliées d'un facteur 2,6 et 8,8 respectivement entre le milieu aqueux et huileux.

La stabilité de ces ferrofluides huileux soumis à plusieurs cycles d'induction magnétiques a également été évaluée.

Les dispersions huileuse et aqueuse de FF2 ont fait l'objet de 4 cycles successifs d'échauffement/refroidissement ($\Delta T$ > 10K, soit de 37°C à plus de 47°C) sous induction magnétique pour 2 couples charrps fréquences différents (2 cycles à 344,5 kHz, 16,23 kA.m$^{-1}$ suivi de 2 cycles à 473,5 kHz, 13,36 kA.m$^{-1}$).

Les dispersions huileuse et aqueuse de FF3 ont fait l'objet de 2 cycles successifs d'échauffement/refroidissement ($\Delta T$ > 40K, soit de 37°C à plus de 77°C) de 2 cycles d'induction magnétique pour un ccuple champ fréquence de 473,5 kHz, 13,36 kA.m$^{-1}$.

Aux figures 6A, 6B et 6Csont représentés respectivement les profils de température des dispersions de nanoparticules FF2 et FF3 dispersées dans l'eau (5 g/kg) et des mêmes nanoparticules fonctionnalisées par la DOPA (pour des densités de recouvrement théorique respectifs de 1,29 et 1,10 molécules/nm$^2$, condition acide, exemples 2.1.3 et 2.1.4) dans le Miglyol M840 (5 g/kg) lors de l'application du champ magnétique alternatif. A la figure 6A, 2 cycles de chauffage sous induction à 473,5 kHz, 13,36 kA.m$^{-1}$ et refroidissement ($T_0$ = 37°C) sont réalisés. A la figure 6B, 2 cycles de chauffage sous induction à 344,5 kHz, 16,23 kA.m$^{-1}$ et refroidissement ($T_0$ = 37°C) sont réalisés. A la figure 6C, deux cyclesde chauffage sous induction à 473,5 kHz, 13,36 kA.m$^{-1}$ et refroidissement ($T_0$ = 37°C) sont réalisés.

Les valeurs de SAR mesurées pour ces dispersions aqueuses et huileuses sont détaillées dans le tableau 11 suivant.

[Table 11]

| | Couple champ fréquence appliqué | SAR 1er cycle (W/g) | SAR 2ème cycle (W/g) | ΔT (°C) en 10s 1er cycle | ΔT (°C) en 10s 2ème cycle |
|---|---|---|---|---|---|
| FF2 $H_2O$ | 344 kHz, 16,23 $kA.m^{-1}$ | 92 | 100,3 | 1,1 | 1,2 |
| FF2 M840 | | 284 | 300 | 7,1 | 7,5 |
| FF2 $H_2O$ | 473,5 kHz, 13,36 $kA.m^{-1}$ | 83,6 | 83,6 | 1,0 | 1,0 |
| FF2 M840 | | 220 | 252 | 5,5 | 6,3 |
| FF3 $H_2O$ | | 291 | 298 | 3,4 | 3,5 |
| FF3 M840 | | 2411 | 2800 | 39,1 | 43,4 |

Les valeurs de SAR à 37°C des nanoparticules magnétiques issues des dispersions FF2 sont multipliées environ d'un facteur 3 respectivement entre le milieu aqueux et huileux pour les deux conditions champ fréquence 344 kHz, 16,23 $kA.m^{-1}$ et 473,5 kHz, 13,36 $kA.m^{-1}$. Pour un temps d'induction de 10 secondes, la température de la dispersion aqueuse FF2 augmente d'environ 1°C tandis que pour les mêmes nanoparticules issues de FF2 fonctionnalisées par la DOPA et dispersées dans le Miglyol M840®, l'augmentation de température est environ 6 à 7 fois supérieure.

Les valeurs de SAR à 37°C des nanoparticules magnétiques issues des dispersions FF3 sont multipliées environ d'un facteur 8,3 à 9,4 entre le milieu aqueux et huileux pour les conditions champ fréquence 473,5 kHz, 13,36 $kA.m^{-1}$. Pour un temps d'induction de 10 secondes, la température de la dispersion aqueuse FF3 augmente d'environ 3,5°C tandis que pour les mêmes nanoparticules issues de FF3 fonctionnalisées par la DOPA et dispersées dans le Miglyol M840®, l'augmentation de température est environ 11 à 12 fois supérieure. Les ferrofluides huileux peuvent donc potentiellement permettre de réduire drastiquement les temps de traitement sous induction.

La réalisation de plusieurs cycles de chauffage par induction met en évidence que les dispersions supportent l'augmentation de température locale des nanoparticules sans modification des propriétés des nanoparticules et de la phase huileuse. Il n'y a pas de dégradation des molécules de phospholipide en surface des nanoparticules. Cette propriété peut s'avérer intéressante dans la perspective de répondre à la nécessité de multiplier les séquences de traitement d'hyperthermie magnéto-induite.

Les valeurs de SAR ont également été mesurées en faisant varier le volume de dispersion des nanoparticules FF3 (5 g/Kg) de 500 μL à 1 μL, dans l'eau et dans l'huile (Miglyol M840®) à partir des profils de température représentés en figures 7A, 7B et 7C.

Le pouvoir thermogène des dispersions ainsi que la valeur plateau de température diminuent avec le volume des dispersions en raison des pertes thermiques qui deviennent de plus en plus prépondérantes avec l'augmentation du rapport surface sur volume. Pour les dispersions huileuses, les pertes thermiques sont beaucoup plus faibles par rapport au ferrofluide aqueux en raison de leur plus faible conductivité thermique et à l'absence de phénomène endothermique comme l'évaporation. Pour des volumes de 200 et 500 μL, le pouvoir chauffant est toujours aussi élevé, 9 fois supérieur à celui des dispersions aqueuses, et aucun palier de température ne peut être observé pour ces conditions de mesures. Des paliers de température de 85° C et 75°C sont atteints assez rapidement (2-3 minutes) respectivement pour les volumes de 50 et 10 μL du fait des valeurs de SAR encore très élevées. Les valeurs plateaux et surtout les valeurs de SAR restent bien supérieures, d'un facteur d'environ 5 à 10, à celles des dispersions aqueuses de FF3. La dispersion FF3 dans le Miglyol M840® montre ainsi des valeurs de SAR très élevées même pour des volumes faibles jusqu'à 10 μL.

La valeur de SAR pour un volume de 1μL est mesurée en déposant directement le ferrofluide au bout de la sonde. La température initiale est alors celle de la pièce (T0 = 25°C). La SAR de 65 W/g estseulement mesurable pour la dispersion de FF3 dans le Miglyol M840®. L'évaporation de l'eau se manifestant par une diminution de la température du milieu de 7°C est trop rapide pour mesurer la SAR de la dispersion FF3 aqueuse pour ce volume.

La figure 8 représente le profil cinétique de température pour un volume de 1 μL de dispersion FF3 dispersées dans le Miglyol M840® utilisée pour les expérimentations animales de thermo-ablation de tumeurs par hyperthermie magnétique présentées en exemple 6. La dispersion de concentration massique en oxyde de fer $Fe_2O_3$ est de 300 g/L ($T_0$=25°C, température ambiante de la pièce) présente un profil cinétique d'échauffement montrant une élévation de température de 75°C en 20 s pour une valeur de SARde 53 W/g. Cette dispersion concentrée à 300 g/L permet des élévations de température locale très rapide et ce même pour des volumes très faibles, de l'ordre du μL, ce qui peut contribuer à améliorer la précision du traitement dans le cadre des thermo-ablations de tumeurs de faibles volumes.

**Exemple 5** : **Préparation de nanoémulsions à partir du ferrofluide huileux**

Les nanoparticules magnétiques FF1 fonctionnalisées par de la DOPA obtenues au point 2.1.1 pour une densité de recouvrement de 1,12 molécules/$nm^2$ sont dispersées dans la phase huileuse constituée de Miglyol 840. La phase huileuse contenant les nanoparticules d'oxyde de fer dispersées est soit utilisée seule directement et constitue alors 100% de la phase huileuse de la nanoémulsion, soit peut être préalablement diluée dans du Miglyol 840 pour constituer 1/3 ou 2/3 de la masse totale de la phase lipophile de la nanoémulsion.

Les nanoémulsions sont préparées en mélangeant éventuellement la phase lipophile (Miglyol 840) avec le ferrofluide huileux afin d'obtenir un mélange homogène dans lequel est dispersé à chaud (70°C) lalécithine d'oeuf E80. La phase aqueuse préalablement chauffée à la même température est mélangée au co-surfactant (polysorbate 80). L'émulsification et l'homogénéisation est obtenue en une seule étape par inversion de phase en utilisant un sonicateur pendant 10 minutes. Après obtention de la nanoémulsion, celle-ci reste stable après abaissement de la température, notamment à une température de 20°C.

Des exemples de compositions de nanoémulsions 1 à 5 sont détaillés dans les tableaux 12 à 16 ci-dessous, dans lesquels les pourcentages sont des pourcentages massiques par rapport au poids total de la nanoémulsion.

[Table 12]

| Nanoémulsion 1 | % |
|---|---|
| Ferrofluide FF1-DOPA (60 mg/mL) en dispersion dans Miglyol 840 | 20 |
| Miglyol 840 | 0 |
| Lécithine d'œuf E80 | 1,2 |
| Polysorbate 80 | 2 |
| Eau | qs 100 |

[Table 13]

| Nanoémulsion 2 | % |
|---|---|
| Ferrofluide FF1-DOPA (60 mg/mL) en dispersion dans Miglyol 840 | 30 |
| Miglyol 840 | 0 |
| Lécithine d'œuf E80 | 1,8 |
| Polysorbate 80 | 2 |
| Eau | qs 100 |

[Table 14]

| Nanoémulsion 3 | % |
|---|---|
| Ferrofluide FF1-DOPA (60 mg/mL) en dispersion dans Miglyol 840 | 35 |
| Miglyol 840 | 0 |
| Lécithine d'œuf E80 | 2,1 |
| Polysorbate 80 | 2 |
| Eau | qs 100 |

[Table 15]

| Nanoémulsion 4 | % |
|---|---|
| Ferrofluide FF1-DOPA (60 mg/mL) en dispersion dans Miglyol 840 | 20 |
| Miglyol 840 | 10 |
| Lécithine d'œuf E80 | 1,8 |
| Polysorbate 80 | 2 |
| Eau | qs 100 |

[Table 16]

| Nanoémulsion 5 | % |
|---|---|
| Ferrofluide FF1-DOPA (60 mg/mL) en dispersion dans Miglyol 840 | 10 |
| Miglyol 840 | 20 |
| Lécithine d'œuf E80 | 1,8 |
| Polysorbate 80 | 2 |
| Eau | qs 100 |

Les mesures de potentiel zêta (PZ) sont réalisées par dilution de l'échantillon au 1/1000eme dans de l'eau désionisée. La valeur du PZ est déterminée par électrophorèse et détection par laser Doppler à l'aide d'un appareil Zetasizer Nano ZS (Malvern Instruments SA, Worcestershier, UK). Les caractéristiques granulométriques (diamètre hydrodynamique moyen, indice de polydispersité (PDI) et potentiel zêta (PZ) des exemples sont données dans le tableau 17 suivant :

[Table 17]

| | Nanoémulsion 1 | Nanoémulsion 2 | Nanoémulsion 3 | Nanoémulsion 4 | Nanoémulsion 5 |
|---|---|---|---|---|---|
| Diamètre moyen (nm) | 181,2± 0,6 | 187,6 ± 2,4 | 188,3 ± 2,3 | 183,7 ± 5,0 | 191,5 ± 0,4 |
| PDI | 0,112 | 0,119 | 0,121 | 0,125 | 0,101 |
| PZ (mV) | -31,8 ± 2,4 | -33,8 ± 0,7 | -40 ± 2,5 | -32,2 ± 0,8 | -36,7 ±0,3 |

Le pouvoir thermogène des nanoémulsions 1 et 4 présentant la même concentration en oxyde de fer du ferrofluide FF1 ($CFe_2O_3$=12 g/L, $T_0$ = 37°C) sous induction à 473,5 KHz, 13,36 kA/m est comparé à la figure 9.

A concentration égale en ferrofluide mais avec un pourcentage de phase huileuse plus important, l'échauffement est amélioré (comparaison nanoémulsions 1 et 4).

**Exemple 6** : **thermoablation de tumeurs sous-cutanées par hyperthermie magnétique**

L'efficacité du ferrofluide huileux selon l'exemple 2.1.4, composé de nanoparticules magnétiques FF3 fonctionnalisées par de la DOPA dans du Miglyol M840®, pour la thermoablation par hyperthermie magnétique de tumeurs chez la souris a été évaluée.

Afin de suivre la distribution du produit dans le milieu tumoral, un fluorophore lipophile émettant dans le proche infrarouge a été préalablement incorporé dans le ferrofluide huileux : le iodure de 1,1'-dioctadecyl-3,3,3',3'-tetramethylindotricarbocyanine (DiR). Le maximum d'émission de ce fluorophore se situe à 780 nm.

Les souris testées sont des souris B6 Albinos (B6N-Tyrc-Brd/BrdCrCrl) portant une tumeur implantée en sous-cutané RM1-CMV-LucF au niveau d'une patte. Les volumes tumoraux ont été estimés à partir des dimensions L et l mesurées au pied à coulisse digital et calculés à partir de la formule de Feldman : Volume = $\pi/6 \times f \times (L \cdot l)^{3/2}$ avec $f$ = 1,58 pour les souris femelles. Les différents volumes ont ainsi été estimés autour de 170 $\pm$ 20 $mm^3$ selon les spécimens.

Compte tenu des volumes tumoraux, les expériences ont été réalisées en procédant à des microinjections intratumorales de ferrofluide huileux de concentration en oxyde de fer de 300 µg/µL. Les ferrofluides huileux ont été injectés directement dans la tumeur. Les injections ont été réalisées à l'aide d'une seringue Hamilton de 10 µL équipée d'une aiguille biseautée (gauge de 26), sous anesthésie à l'isoflurane puis les souris sont placées dans un lit chauffant

sous les bobines d'induction. Seul un traitement par induction à 473,5 kHz et 13,36 $kA.m^{-1}$ pendant 15 minutes a été réalisé pour chaque souris.

Sur une première expérience, une injection intratumorale de 2 $\mu$L de ferrofluide huileux FF3 (300 $\mu$g/$\mu$L en oxyde de fer, soit une masse de 600 $\mu$g) est réalisée à une profondeur de 2,5 mm sur un nouveau spécimen séparée en deux fois 1 $\mu$L avec une minute d'attente entre les deux injections (réalisées en un même point d'injection). A l'issue de la dernière injection, un temps d'attente d'une minute est à nouveau appliqué avant le retrait de l'aiguille. La dose thermique dissipée par les nanoparticules dans le volume tumoral (Qv) peut en première approximation être calculée selon la formule : $Q_V = m_P (Fe_2O_3) \times SAR(1\mu L) \times t / V_{tumeur}$. Dans ces conditions opératoires, la dose thermique Qv = 0,17 $J/mm^3$. Les images de bioluminescence et de fluorescence ont été réalisées suite à l'injection avant le traitement par induction et 24h après. Tel qu'illustré en figure 10A, une zone de thermoablation peut être clairement observée au centre de la tumeur. Pendant l'application du champ magnétique alternatif, l'écart de température mesurée par la caméra thermique était de 3°C. La croissance tumorale, mesurée par intégration de la bioluminescence 24 h après, se trouve ralentie. Les images de fluorescence *ex vivo* après résection de la tumeur montre la localisation de la zone d'injection du ferrofluide. L'image de bioluminescence *ex vivo* indique une perte de viabilité des cellules cancéreuses à l'endroit où le ferrofluide a été injecté (figure 10B).

Sur une deuxième expérience, des injections multiples de trois fois 1 $\mu$L de ferrofluide huileux FF3 (300 $\mu$g/$\mu$L, soit une masse de 900 $\mu$g) ont été réalisées à 2,5 mm de profondeur, réparties sur trois endroits distincts de la tumeur (figure 11A). Un temps d'attente d'une minute a été appliqué avant retrait de l'aiguille pour chaque injection. Dans ces conditions opératoires, la dose thermique Qv = 0,25 $J/mm^3$. 24 h après l'application du champ magnétique alternatif ($\Delta T \approx 20°C$), on peut aisément distinguer les zones d'ablation de la tumeur aux voisinages des zones d'injections du ferrofluide. Les mesures *ex vivo* confirment l'ablation partielle de la tumeur au niveau des zones traitées (figure 11B).

## Revendications

1. Ferrofluide huileux biocompatible comprenant des nanoparticules magnétiques à base d'oxyde de fer et une phase huileuse comportant au moins un ester d'acide gras, **caractérisé en ce que** lesdites nanoparticules magnétiques sont fonctionnalisées en surface par des molécules de un ou plusieurs phospholipides.

2. Ferrofluide huileux biocompatible selon la revendication 1 comprenant des nanoparticules magnétiques à base d'oxyde de fer et une phase huileuse comportant au moins un ester d'acide gras, lesdites nanoparticules magnétiques à base d'oxyde de fer formant une dispersion colloïdale dans ladite phase huileuse à partir d'une température appartenant à la gamme allant de 20 à 80°C, **caractérisé en ce que** lesdites nanoparticules magnétiques sont fonctionnalisées en surface par des molécules de un ou plusieurs phospholipides qui ne recouvrent pas totalement la surface des nanoparticules magnétiques à base d'oxyde de fer, et, en particulier, qui assurent un taux de recouvrement de la surface des nanoparticules magnétiques à base d'oxyde de fer tel que le ou les esters d'acide gras présent(s) dans la phase huileuse ont accès à la surface des nanoparticules magnétiques à base d'oxyde de fer.

3. Ferrofluide huileux selon la revendication 1 ou 2 **caractérisé en ce qu'**il est exempt d'eau et / ou exempt de tensioactif.

4. Ferrofluide huileux selon l'une des revendications 1 à 3, les molécules de phospholipide(s) assurent un recouvrement de 19 à 76 %, de préférence de 29 à 76 %, et préférentiellement de 34 à 50%, de la surface des nanoparticules magnétiques à base d'oxyde de fer.

5. Ferrofluide huileux selon l'une quelconque des revendications précédentes, **caractérisé en ce que** la densité surfacique de greffage des nanoparticules magnétiques par les molécules de phospholipide(s) sur les appartient à la gamme allant de 0,32 molécule/$nm^2$ à 1,22 molécules/$nm^2$, de préférence de 0,48 molécule/$nm^2$ à 1,22 molécules/$nm^2$ et préférentiellement de 0,56 molécule/$nm^2$ à 0,79 molécules/$nm^2$.

6. Ferrofluide huileux selon l'une quelconque des revendications précédentes, **caractérisé en ce que** le ou les phospholipides contiennent au moins une chaîne grasse, de préférence 2 chaînes grasses, en particulier des chaînes hydrocarbonées, saturées ou mono- ou polyinsaturées, ramifiées ou de préférence linéaires, en C6-C30 et de préférence en C8-C24, voire en C10-C22, et en particulier en C18.

7. Ferrofluide huileux selon l'une quelconque des revendications précédentes, **caractérisé en ce que** la phase huileuse comprend au moins 70% en masse d'ester(s) d'acide gras, de préférence la phase huileuse comprend de 80% à 95% en masse d'ester(s) d'acide gras, par rapport à la masse totale de la phase huileuse.

8. Ferrofluide huileux selon l'une quelconque des revendications précédentes, **caractérisé en ce que** le ou les esters d'acide gras de la phase huileuse sont choisis parmi les triglycérides d'acide gras saturés en C6-C12, de préférence en C6-C10, les propylènes glycols d'acide gras saturés en C6-C12, de préférence en C6-C10, utilisés seuls ou en mélange.

9. Ferrofluide huileux selon l'une quelconque des revendications précédentes, **caractérisé en ce que** la teneur en nanoparticules magnétiques appartient à la gamme allant de 0,01% à 50% en masse, de préférence de 0,1% à 10% en masse, par rapport à la masse totale de ferrofluide huileux.

10. Ferrofluide huileux selon l'une quelconque des revendications précédentes, **caractérisé en ce que** les nanoparticules magnétiques sont sous forme de sphéroïde, de polyèdre tel que les nanocubes, les bipyramides ou les nanoétoiles, de plaquette, de nanobâtonnet, de nanodisque, ou de nanofleur.

11. Ferrofluide huileux selon l'une quelconque des revendications précédentes, **caractérisé en ce que** le ou les phospholipides présente(nt) une tête polaire $-O(O)P(OH)O^-$ et, est(sont), de préférence, choisi(s) parmi les sels de l'acide 1,2-dioléoyl-sn-glycéro-3-phosphatidique et de l'acide 1,2-distéaroyl-sn-glycéro-3-phosphatidique.

12. Ferrofluide huileux selon l'une quelconque des revendications précédentes comprenant en outre un principe actif lipophile, en particulier choisi parmi les anticancéreux, tels que le Paclitaxel, le Docetaxel, ou la Carmustine.

13. Procédé de préparation d'un ferrofluide huileux selon l'une quelconque des revendications précédentes comprenant les étapes successives suivantes :

    a- disposer d'une dispersion aqueuse de nanoparticules magnétiques à base d'oxyde de fer, dans un solvant aqueux qui peut être de l'eau ou un mélange eau/solvant(s) miscible(s) à l'eau,
    b - éliminer le solvant aqueux de la dispersion aqueuse de nanoparticules magnétiques,
    c - obtenir un sol colloïdal de nanoparticules magnétiques par ajout de solvant ou mélange de solvants organiques volatils (**S2**),
    d - fonctionnaliser en surface lesdites nanoparticules magnétiques du sol colloïdal par des molécules d'au moins un phospholipide,
    e - éliminer ledit ou lesdits solvants organiques volatils (**S2**) et disperser les nanoparticules magnétiques fonctionnalisées dans une phase huileuse comportant au moins un ester d'acide gras.

14. Procédé de préparation d'un ferrofluide huileux selon la revendication 13, comprenant une étape c2, ultérieure à l'étape c et antérieure l'étape d, d'ajout d'acide.

15. Médicament comprenant un ferrofluide huileux selon l'une quelconque des revendications 1 à 12 ou un ferrofluide huileux obtenu selon le procédé de préparation selon l'une quelconque des revendications 13 ou 14.

16. Nanoémulsion huile-dans-eau comprenant de 10% à 30% en masse d'un ferrofluide huileux selon l'une quelconque des revendications 1 à 12 ou d'un ferrofluide huileux obtenu selon le procédé de préparation selon l'une quelconque des revendications 13 ou 14, une phase aqueuse, et au moins un tensioactif.

17. Produit de contraste comprenant un ferrofluide huileux biocompatible selon l'une quelconque des revendications 1 à 12.

18. Ferrofluide huileux biocompatible selon l'une quelconque des revendications 1 à 12 ou ferrofluide huileux biocompatible obtenu selon le procédé selon la revendication 13 ou 14, pour son utilisation lors d'un traitement du cancer par hyperthermie déclenchée sous induction magnétique.

**Patentansprüche**

1. Biokompatibles öliges Ferrofluid, umfassend magnetische Nanopartikel auf Eisenoxidbasis und eine ölige Phase, die mindestens einen Fettsäureester beinhaltet, **dadurch gekennzeichnet, dass** die magnetischen Nanopartikel durch Moleküle eines oder mehrerer Phospholipide oberflächenfunktionalisiert sind.

2. Biokompatibles öliges Ferrofluid nach Anspruch 1, umfassend magnetische Nanopartikel auf Eisenoxidbasis und

eine ölige Phase, die mindestens einen Fettsäureester beinhaltet, wobei die magnetischen Nanopartikel auf Eisenoxidbasis eine kolloidale Dispersion in der öligen Phase ab einer Temperatur bilden, die in einem Bereich von 20 bis 80 °C liegt, **dadurch gekennzeichnet, dass** die magnetischen Nanopartikel durch Moleküle eines oder mehrerer Phospholipide oberflächenfunktionalisiert sind, welche die Oberfläche der magnetischen Nanopartikel auf Eisenoxidbasis nicht vollständig abdecken und die insbesondere eine Abdeckungsrate der Oberfläche der magnetischen Nanopartikel auf Eisenoxidbasis derart sicherstellen, dass der oder die in der öligen Phase vorhandene(n) Fettsäureester Zugang zu der Oberfläche der magnetischen Nanopartikel auf Eisenoxidbasis hat/haben.

**3.** Öliges Ferrofluid nach Anspruch 1 oder 2, **dadurch gekennzeichnet, dass** es frei von Wasser und/oder frei von Tensid ist.

**4.** Öliges Ferrofluid nach einem der Ansprüche 1 bis 3, wobei die Phospholipidmoleküle eine Abdeckung von 19 bis 76 %, bevorzugt 29 bis 76 % und vorzugsweise 34 bis 50 %, der Oberfläche der magnetischen Nanopartikel auf Eisenoxidbasis sicherstellen.

**5.** Öliges Ferrofluid nach einem der vorhergehenden Ansprüche, **dadurch gekennzeichnet, dass** die Oberflächenpfropfdichte der magnetischen Nanopartikel mit den Phospholipidmolekülen darauf in dem Bereich von 0,32 Molekülen/$nm^2$ bis 1,22 Molekülen/$nm^2$, bevorzugt 0,48 Molekülen/$nm^2$ bis 1,22 Molekülen/$nm^2$ und vorzugsweise 0,56 Molekülen/$nm^2$ bis 0,79 Molekülen/$nm^2$, liegt.

**6.** Öliges Ferrofluid nach einem der vorhergehenden Ansprüche, **dadurch gekennzeichnet, dass** das oder die Phospholipid(e) mindestens eine Fettkette, bevorzugt 2 Fettketten, insbesondere gesättigte oder einfach oder mehrfach gesättigte, verzweigte oder bevorzugt lineare C6-C30- und bevorzugt C8-C24-, sogar C10-C22-und insbesondere C18-Kohlenwasserstoffketten enthält/enthalten.

**7.** Öliges Ferrofluid nach einem der vorhergehenden Ansprüche, **dadurch gekennzeichnet, dass** die ölige Phase bezogen auf die Gesamtmasse der öligen Phase mindestens 70 Masse-% Fettsäureester umfasst, die ölige Phase bevorzugt 80 bis 95 Masse-% Fettsäureester umfasst.

**8.** Öliges Ferrofluid nach einem der vorhergehenden Ansprüche, **dadurch gekennzeichnet, dass** der oder die Fettsäureester der öligen Phase ausgewählt ist/sind aus gesättigten C6-C12-, bevorzugt C6-C10-Fettsäuretriglyceriden, gesättigten C6-C12-, bevorzugt C6-C10-Fettsäurepropylenglykolen, die allein oder im Gemisch verwendet werden.

**9.** Öliges Ferrofluid nach einem der vorhergehenden Ansprüche, **dadurch gekennzeichnet, dass** der Gehalt an magnetischen Nanopartikeln bezogen auf die Gesamtmasse des öligen Ferrofluids in dem Bereich von 0,01 bis 50 Masse-%, bevorzugt 0,1 bis 10 Masse-%, liegt.

**10.** Öliges Ferrofluid nach einem der vorhergehenden Ansprüche, **dadurch gekennzeichnet, dass** die magnetischen Nanopartikel in Form einer Kugel, eines Polyeders, wie etwa Nanoröhrchen, Bipyramiden oder Nanosterne, eines Plättchens, eines Nanostäbchens, einer Nanoscheibe oder einer Nanoblume vorliegen.

**11.** Öliges Ferrofluid nach einem der vorhergehenden Ansprüche, **dadurch gekennzeichnet, dass** das oder die Phospholipid(e) einen polaren Kopf $-O(O)P(OH)O^-$ aufweist/aufweisen und bevorzugt ausgewählt ist/sind aus Salzen von 1,2-Dioleoyl-sn-glycero-3-phosphatidsäure und 1,2-Distearoyl-sn-glycero-3-phosphatidsäure.

**12.** Öliges Ferrofluid nach einem der vorhergehenden Ansprüche, ferner umfassend einen lipophilen Wirkstoff, der insbesondere ausgewählt ist aus Krebsmedikamenten, wie etwa Paclitaxel, Docetaxel oder Carmustin.

**13.** Verfahren zur Herstellung eines öligen Ferrofluids nach einem der vorhergehenden Ansprüche, umfassend die folgenden aufeinanderfolgenden Schritte:

a - Bereitstellen einer wässrigen Dispersion von magnetischen Nanopartikeln auf Eisenoxidbasis in einem wässrigen Lösungsmittel, bei dem es sich um Wasser oder ein Gemisch aus Wasser/Lösungsmittel(n), das/die mit Wasser mischbar ist/sind, handeln kann,
b - Abscheiden des wässrigen Lösungsmittels aus der wässrigen Dispersion von magnetischen Nanopartikeln,
c - Erhalten eines kolloidalen Sols von magnetischen Nanopartikeln durch Zusetzen von Lösungsmittel oder eines Gemischs aus flüchtigen organischen Lösungsmitteln (**S2**),
d - Oberflächenfunktionalisieren der magnetischen Nanopartikel des kolloidalen Sols mit Molekülen mindestens

eines Phospholipids,
e - Abscheiden des oder der flüchtigen organischen Lösungsmittel (S2) und Dispergieren der funktionalisierten magnetischen Nanopartikel in einer öligen Phase, die mindestens einen Fettsäureester beinhaltet.

**14.** Verfahren zur Herstellung eines öligen Ferrofluids nach Anspruch 13, umfassend einen Schritt c2, nach dem Schritt c und vor dem Schritt d, des Zusetzens von Säure.

**15.** Medikament, umfassend ein öliges Ferrofluid nach einem der Ansprüche 1 bis 12 oder ein öliges Ferrofluid, das gemäß dem Herstellungsverfahren nach einem der Ansprüche 13 oder 14 erhalten wurde.

**16.** Öl-in-Wasser-Nanoemulsion, umfassend 10 bis 30 Masse-% eines öligen Ferrofluids nach einem der Ansprüche 1 bis 12 oder eines öligen Ferrofluids, das gemäß dem Herstellungsverfahren nach einem der Ansprüche 13 oder 14 erhalten wurde, eine wässrige Phase und mindestens ein Tensid.

**17.** Kontrastmittel, umfassend ein biokompatibles öliges Ferrofluid nach einem der Ansprüche 1 bis 12.

**18.** Biokompatibles öliges Ferrofluid nach einem der Ansprüche 1 bis 12 oder biokompatibles öliges Ferrofluid, das gemäß dem Verfahren nach Anspruch 13 oder 14 erhalten wurde, zur Verwendung bei einer Krebsbehandlung mittels unter magnetischer Induktion ausgelöster Hyperthermie.

## Claims

**1.** A biocompatible oily ferrofluid comprising iron-oxide based magnetic nanoparticles and an oil phase comprising at least one fatty acid ester, **characterized in that** said magnetic nanoparticles are surface functionalized by molecules of one or more phospholipids.

**2.** Biocompatible oily ferrofluid according to claim 1, comprising iron-oxide based magnetic nanoparticles and an oil phase comprising at least one fatty acid ester, said iron-oxide based magnetic nanoparticles forming a colloidal dispersion in said oil phase from a temperature belonging to the range from 20 to 80°C, **characterized in that** said magnetic nanoparticles are surface functionalized by molecules of one or more phospholipids which do not completely cover the surface of the iron-oxide based magnetic nanoparticles and, in particular, which ensure a coverage rate of the surface of the iron-oxide based magnetic nanoparticles such that the fatty acid ester(s) present in the oil phase have access to the surface of the iron-oxide based magnetic nanoparticles.

**3.** Oily ferrofluid according to claim 1 or 2, **characterized in that** it is free of water and/or free of surfactant.

**4.** Oily ferrofluid according to one of claims 1 to 3, wherein the phospholipid molecules ensure a coverage of 19 to 76%, preferably 29 to 76%, and preferentially 34 to 50%, of the surface of iron-oxide based magnetic nanoparticles.

**5.** Oily ferrofluid according to any one of the preceding claims, **characterized in that** the surface density of grafting of the magnetic nanoparticles by phospholipid(s) molecules belongs to the range from 0.32 molecule/$nm^2$ to 1.22 molecules/$nm^2$, preferably 0.48 molecule/$nm^2$ to 1.22 molecules/$nm^2$, and preferentially 0.56 molecule/$nm^2$ to 0.79 molecule/$nm^2$.

**6.** Oily ferrofluid according to any one of the preceding claims, **characterized in that** the phospholipid(s) contain at least one fatty chain, preferably two fatty chains, in particular C6-C30, preferably C8-C24, or even C10-C22, and in particular, C18 hydrocarbonated, saturated or mono- or polyunsaturated, branched or preferably linear hydrocarbon chains.

**7.** Oily ferrofluid according to any one of the preceding claims, **characterized in that** the oil phase comprises at least 70% by mass of fatty acid ester(s), preferably the oil phase comprises 80% to 95% by mass of fatty acid ester(s), relative to the total mass of the oil phase.

**8.** Oily ferrofluid according to any one of the preceding claims, **characterized in that** the fatty acid ester(s) of the oil phase are chosen from C6-C12, preferably C6-C10, saturated fatty acid triglycerides and C6-C12, preferably C6-C10, saturated fatty acid of propylene glycols, used alone or as a mixture.

9. Oily ferrofluid according to any one of the preceding claims, **characterized in that** the magnetic nanoparticle content belongs to the range from 0.01% to 50% by mass, preferably from 0.1% to 10% by mass, relative to the total mass of the oily ferrofluid.

10. Oily ferrofluid according to any one of the preceding claims, **characterized in that** the magnetic nanoparticles are in the shape of a spheroid, a polyhedron such as nanocubes, bipyramids or nanostars, a wafer, a nanorod, a nanodisk or a nanoflower.

11. Oily ferrofluid according to any one of the preceding claims, **characterized in that** the phospholipid(s) have an $-O(O)P(OH)O^-$ polar head and are preferably chosen from salts of 1,2-dioleoyl-sn-glycero-3-phosphatidic acid and 1,2-distearoyl-sn-glycero-3-phosphatidic acid.

12. Oily ferrofluid according to any one of the preceding claims, further comprising a lipophilic active ingredient, in particular chosen from cancer treatment drugs, such as Paclitaxel, Docetaxel or Carmustine.

13. A process for preparing an oily ferrofluid according to any one of the preceding claims comprising the following successive steps:

a - providing an aqueous dispersion of iron-oxide based magnetic nanoparticles in an aqueous solvent which can be water or a mixture of water/solvent(s) miscible with water,
b - eliminating the aqueous solvent from the aqueous dispersion of magnetic nanoparticles,
c - obtaining a colloidal sol of magnetic nanoparticles by addition of solvent or a mixture of volatile organic solvents (S2),
d - surface functionalizing said magnetic nanoparticles of the colloidal sol with molecules of at least one phospholipid,
e - eliminating said volatile organic solvent(s) (**S2**) and dispersing the functionalized magnetic nanoparticles in an oil phase comprising at least one fatty acid ester.

14. Process for preparing an oily ferrofluid according to claim 13, comprising a step c2, after step c and before step d, of adding acid.

15. A medicament comprising an oily ferrofluid according to any one of claims 1 to 12 or an oily ferrofluid obtained according to the preparation process according to any one of claims 13 or 14.

16. An oil-in-water nanoemulsion comprising from 10% to 30% by mass of an oily ferrofluid according to any one of claims 1 to 12 or an oily ferrofluid obtained according to the preparation process according to any one of claims 13 or 14, an aqueous phase and at least one surfactant.

17. A contrast product comprising a biocompatible oily ferrofluid according to any one of claims 1 to 12.

18. A biocompatible oily ferrofluid according to any one of claims 1 to 12 or biocompatible oily ferrofluid obtained according to the process according to claim 13 or 14, for its use in cancer treatment by magnetic-induced hyperthermia.

[Fig. 1]

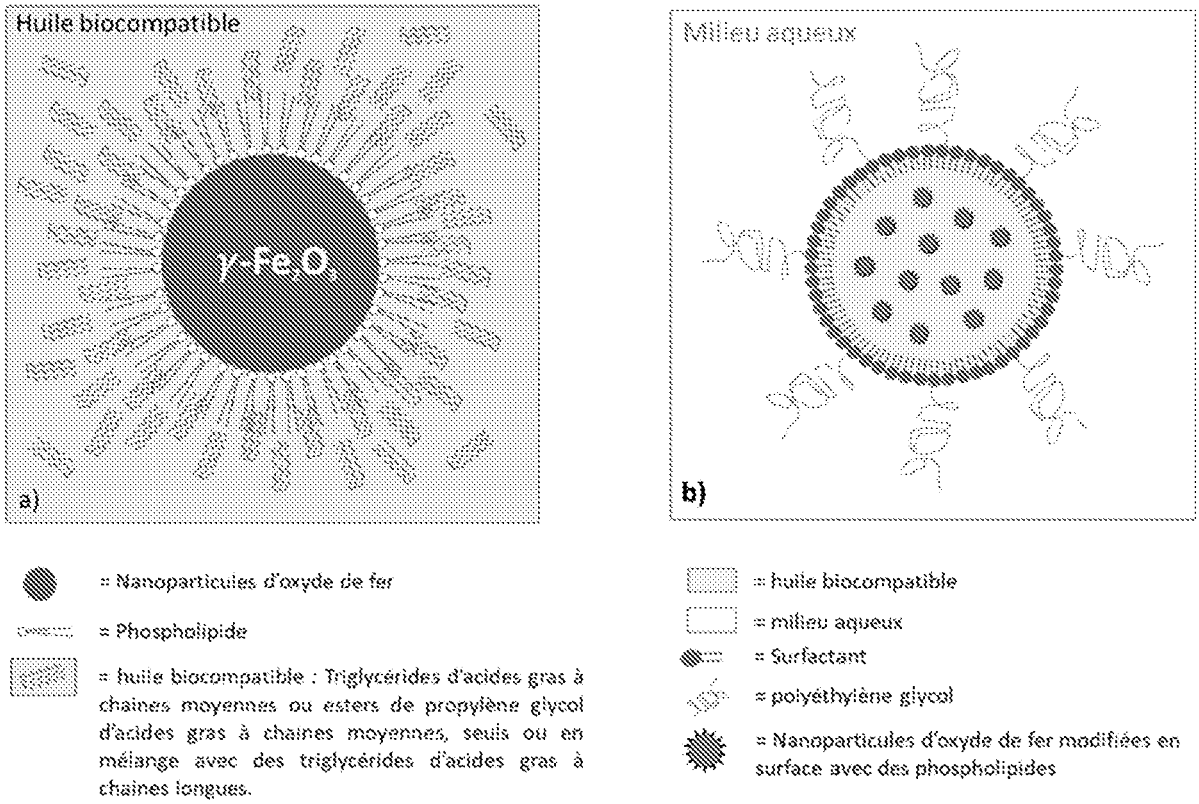
Huile biocompatible
γ-Fe$_2$O$_3$
a)
Milieu aqueux
b)
= Nanoparticules d'oxyde de fer
= Phospholipide
= huile biocompatible : Triglycérides d'acides gras à chaines moyennes ou esters de propylène glycol d'acides gras à chaines moyennes, seuls ou en mélange avec des triglycérides d'acides gras à chaines longues.
= huile biocompatible
= milieu aqueux
= Surfactant
= polyéthylène glycol
= Nanoparticules d'oxyde de fer modifiées en surface avec des phospholipides

[Fig. 2]

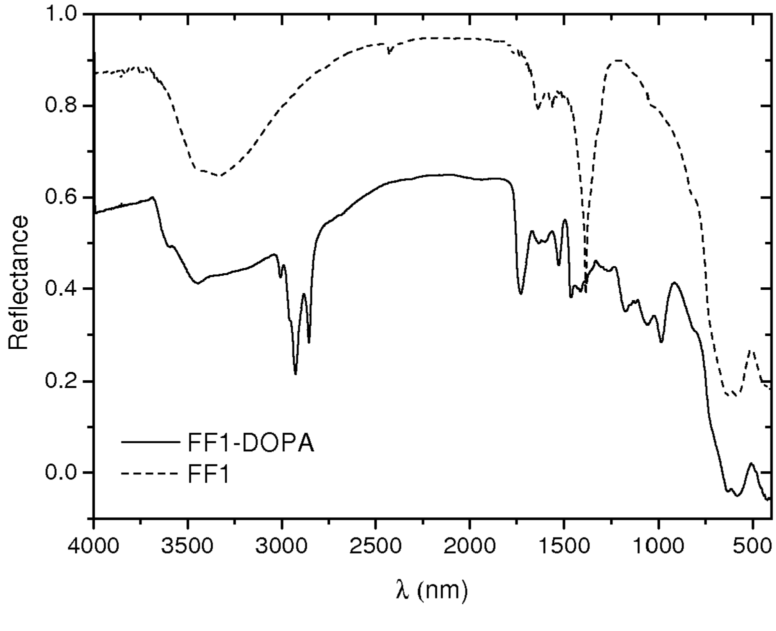
Reflectance
λ (nm)
FF1-DOPA
FF1
1.0
0.8
0.6
0.4
0.2
0.0
4000
3500
3000
2500
2000
1500
1000
500

[Fig. 3]

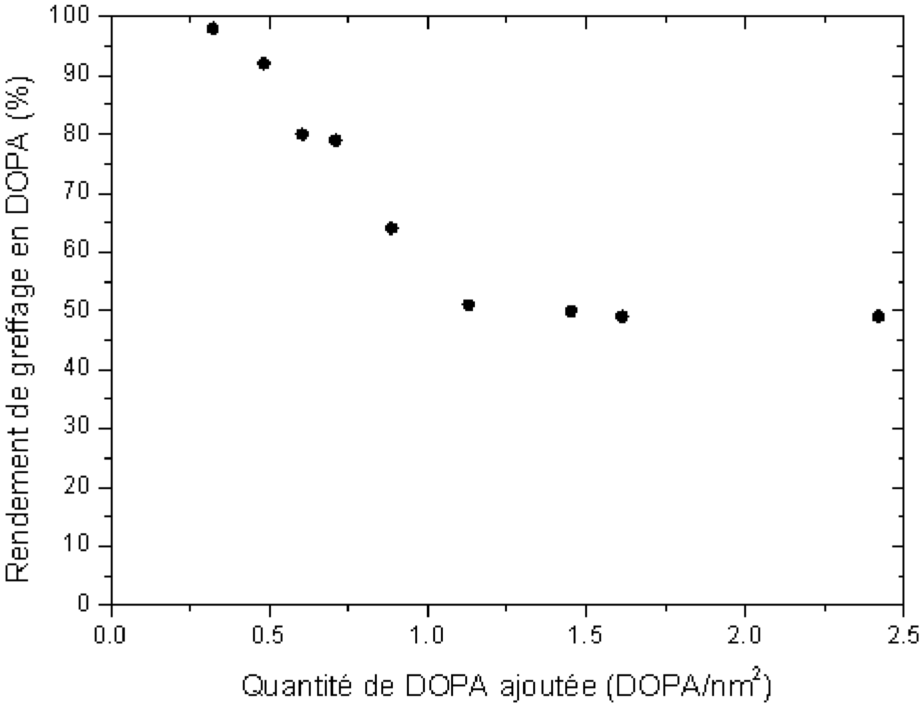
Rendement de greffage en DOPA (%)
0
10
20
30
40
50
60
70
80
90
100
0.0
0.5
1.0
1.5
2.0
2.5
Quantité de DOPA ajoutée (DOPA/nm2)

[Fig. 4]

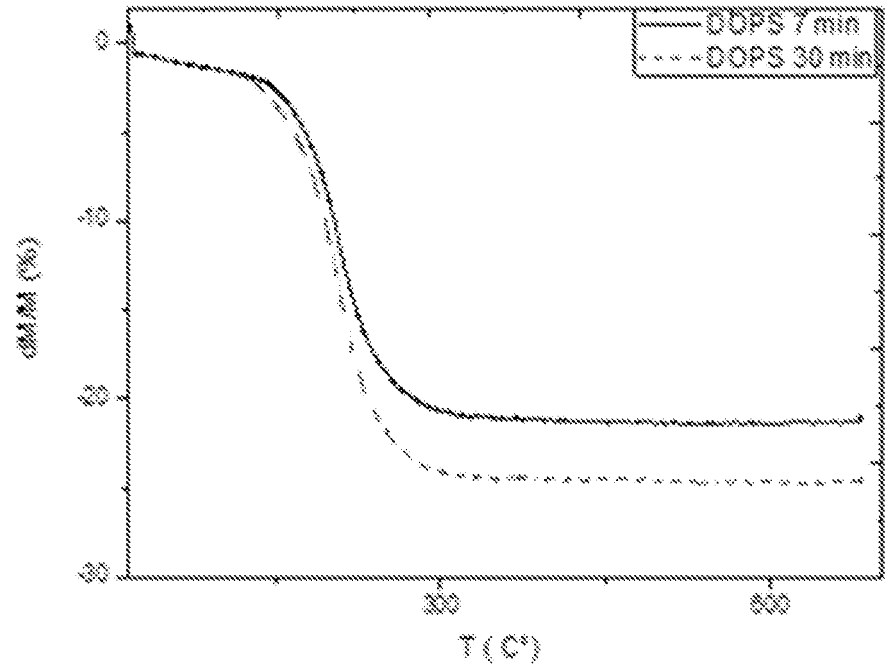
DOPS 7 min
DOPS 30 min
0
-10
-20
-30
300
600
T ( C°)

[Fig. 5 A-C]

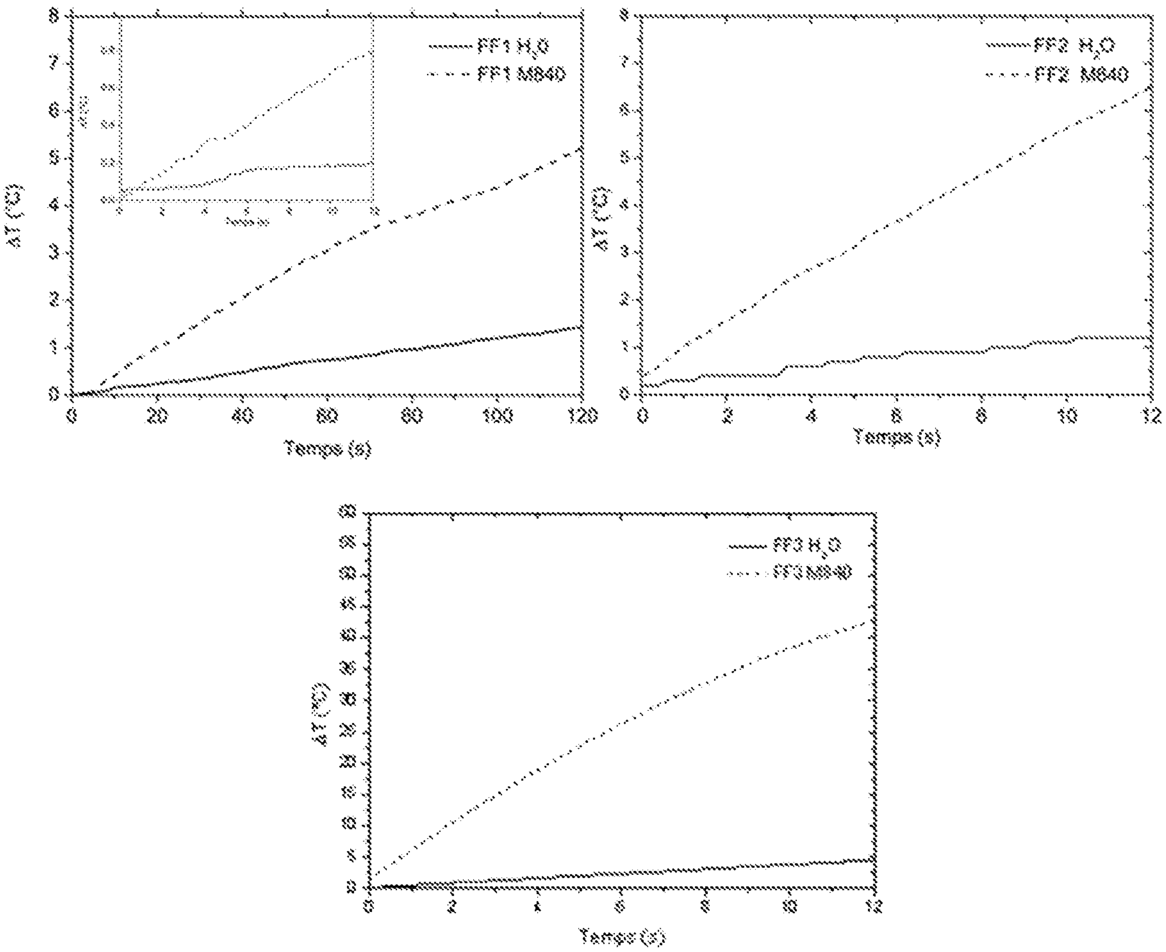


[Fig. 6A-B]

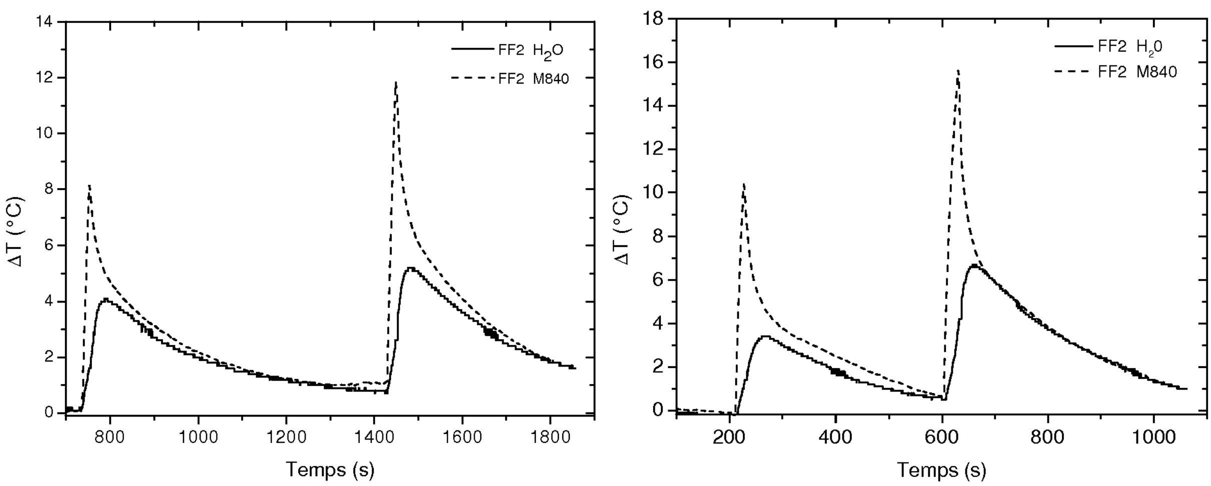

[Fig. 6 C]

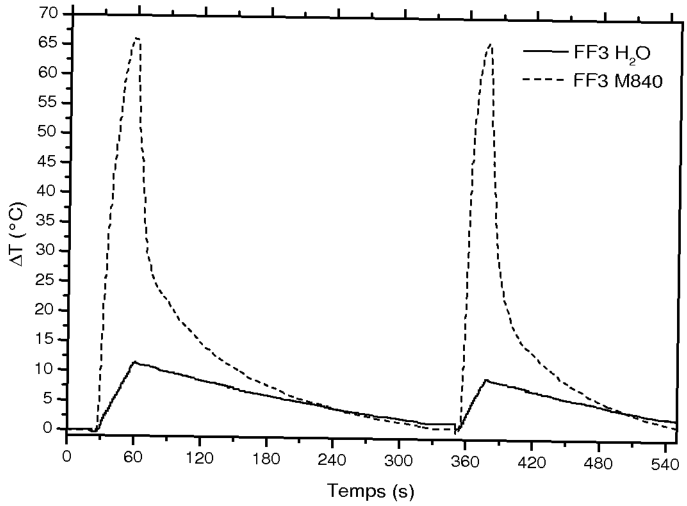


[Fig. 7A-B]

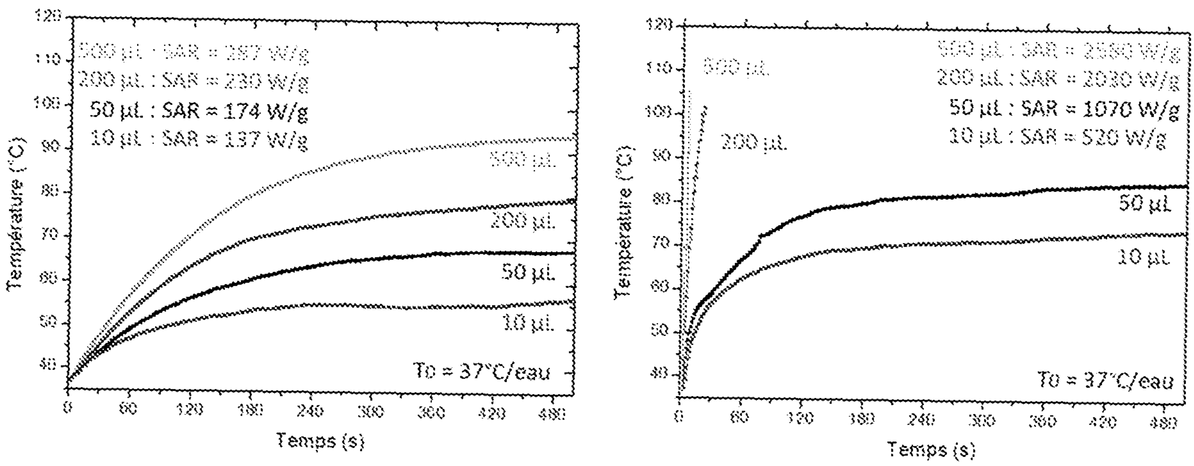

[Fig 7C]

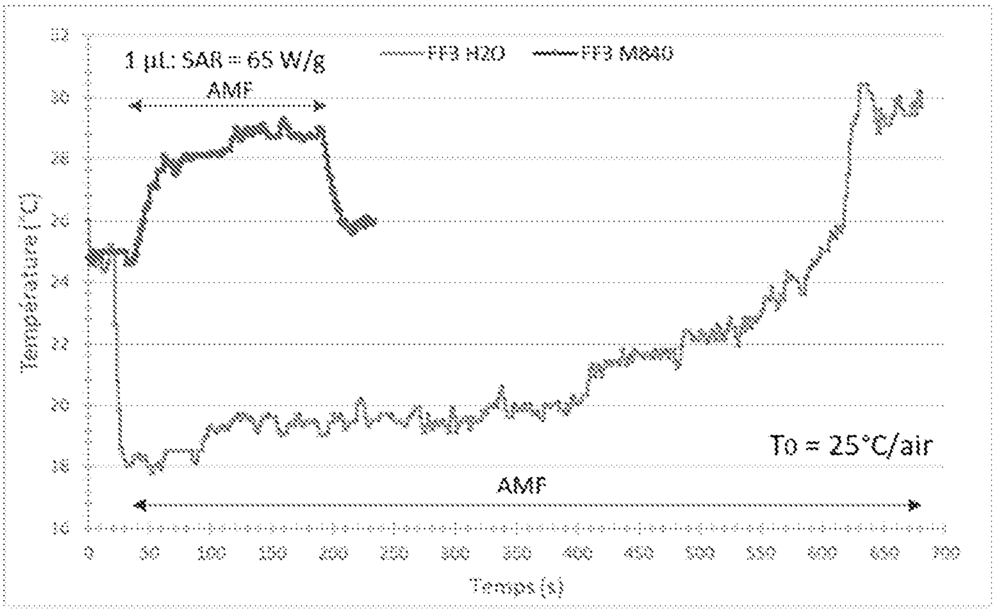
1 µL: SAR = 65 W/g
AMF
FF3 H2O
FF3 M840
Température (°C)
To = 25°C/air
AMF
Temps (s)

[Fig. 8]

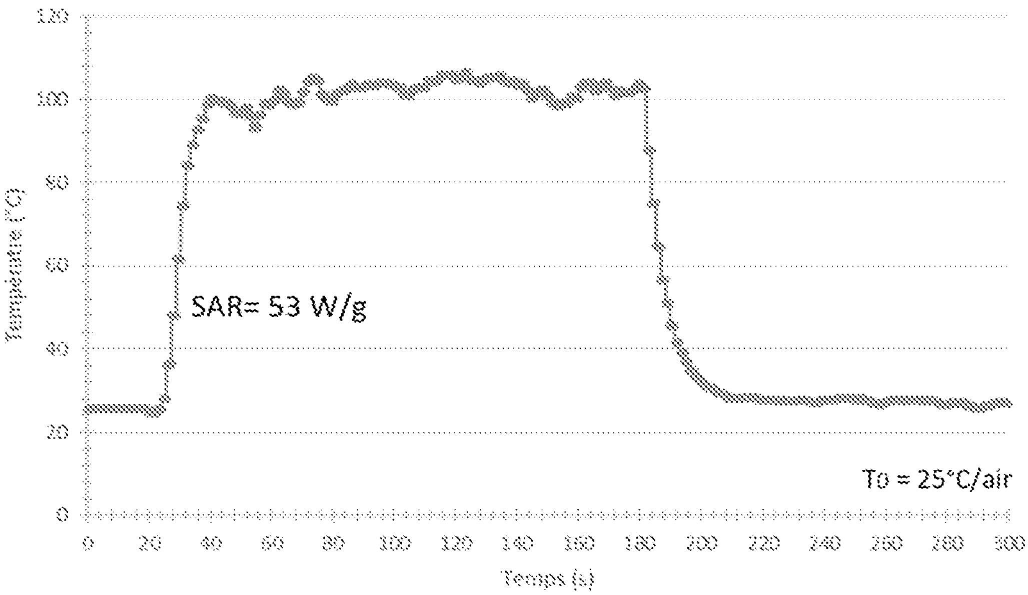
Température (°C)
SAR= 53 W/g
To = 25°C/air
Temps (s)

[Fig. 9]

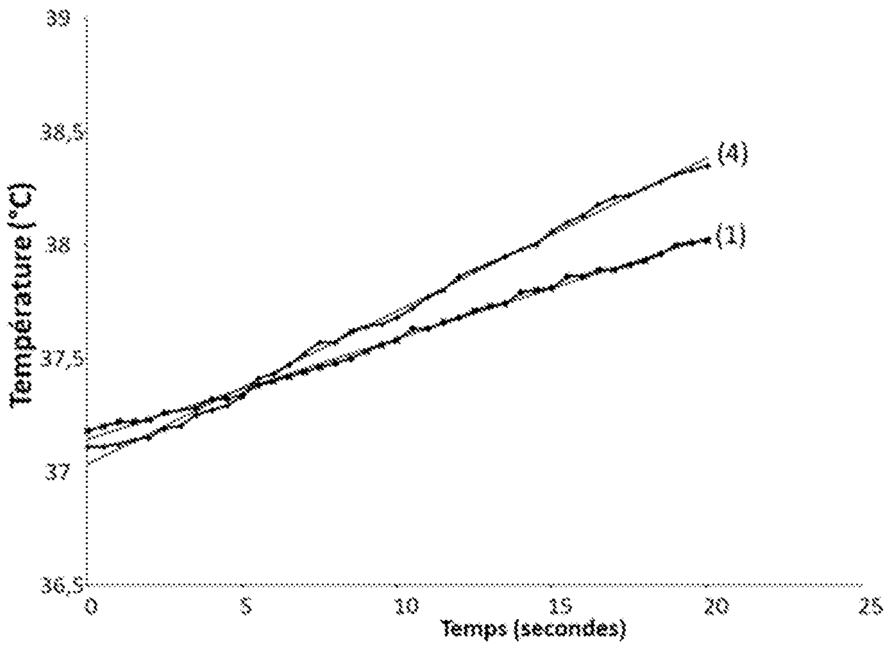
39
38,5
38
37,5
37
36,5
Température (°C)
0
5
10
15
20
25
Temps (secondes)
(4)
(1)

[Fig. 10A]

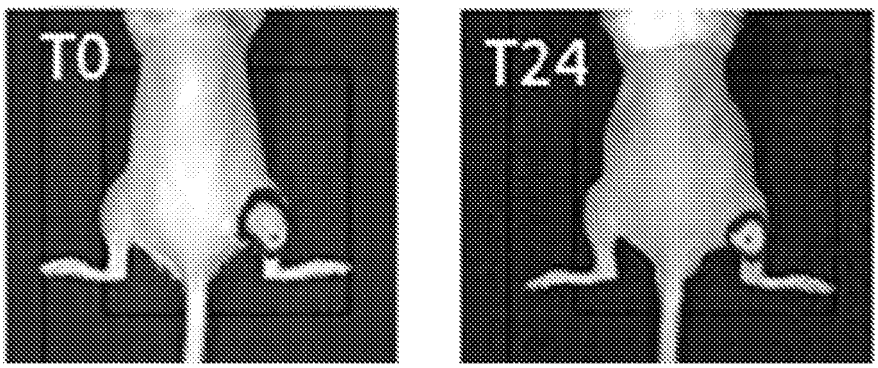
T0
T24

[Fig. 10B]

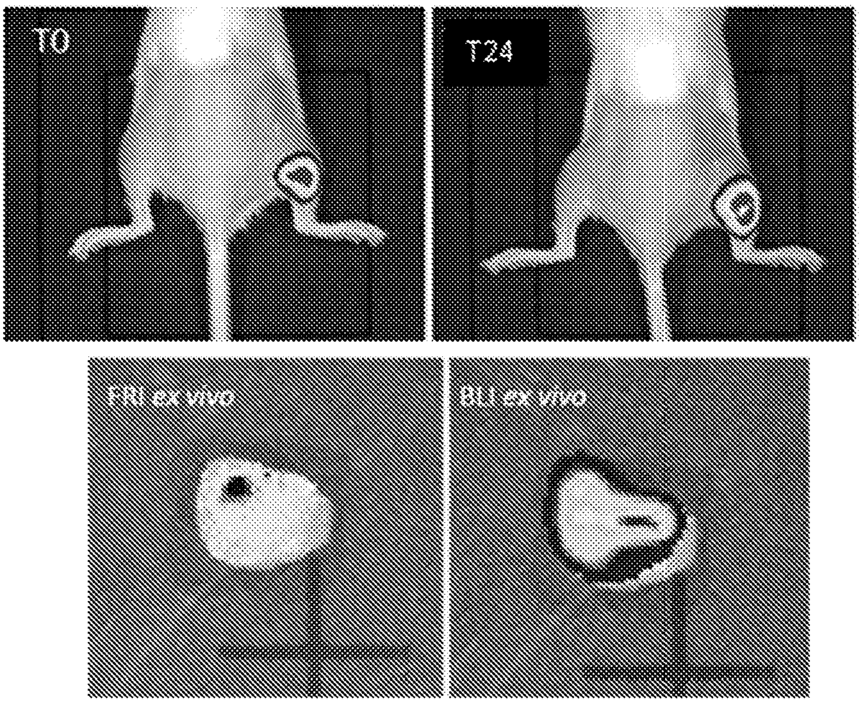


[Fig. 11A-B]

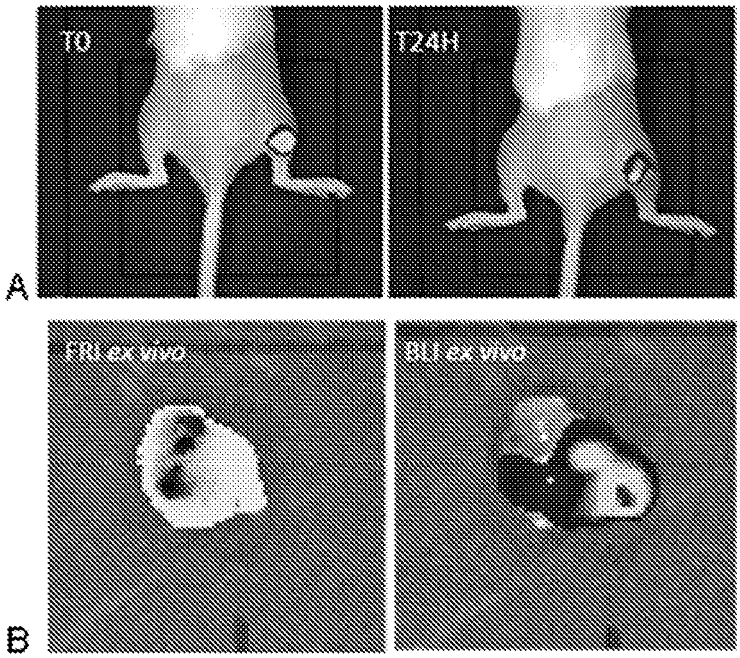

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 3001154